(19) Europäisches Patentamt
European Patent Office
Office européen des brevets

(11) EP 0 854 857 B1

(12) EUROPÄISCHE PATENTSCHRIFT

(45) Veröffentlichungstag und Bekanntmachung des Hinweises auf die Patenterteilung:
**08.03.2000 Patentblatt 2000/10**

(21) Anmeldenummer: **96931789.0**

(22) Anmeldetag: **11.09.1996**

(51) Int Cl.⁷: **C07C 227/32**

(86) Internationale Anmeldenummer:
**PCT/EP96/03984**

(87) Internationale Veröffentlichungsnummer:
**WO 97/10203 (20.03.1997 Gazette 1997/13)**

(54) **VERFAHREN ZUR HERSTELLUNG VON OPTISCH AKTIVEN ALPHA-AMINOSÄUREN UND ALPHA-AMINOSÄURE-DERIVATEN**

METHOD OF PREPARING OPTICALLY ACTIVE ALPHA-AMINO ACIDS AND ALPHA-AMINO ACID DERIVATIVES

PROCEDE DE PRODUCTION D'ALPHA-AMINOACIDES OPTIQUEMENT ACTIFS ET DERIVES D'ACIDES AMINES

(84) Benannte Vertragsstaaten:
**BE CH DE ES FR GB IE LI NL**

(30) Priorität: **11.09.1995 DE 19533617**

(43) Veröffentlichungstag der Anmeldung:
**29.07.1998 Patentblatt 1998/31**

(73) Patentinhaber: **Degussa-Hüls Aktiengesellschaft 60287 Frankfurt am Main (DE)**

(72) Erfinder:
- **ALTENBACH, Hans-Josef D-42119 Wuppertal (DE)**
- **KOTTENHAHN, Matthias D-63579 Freigericht (DE)**
- **MATTHÄUS, Mike D-42327 Wuppertal (DE)**
- **VOGT, Annegret 63450 Hanau (DE)**

(56) Entgegenhaltungen:
**EP-A- 0 542 099**

- **TETRAHEDRON, Bd. 44, Nr. 17, 1988, OXFORD GB, Seiten 5277-92, XP002021578 R. FITZI ET AL.: "Resolution and use in .alpha.-amino acid synthesis of imidazolidinone glycine derivatives" in der Anmeldung erwähnt**
- **JOURNAL OF ORGANIC CHEMISTRY, Bd. 51, Nr. 19, 1986, EASTON US, Seiten 3746-3748, XP002021579 W. H. PEARSON ET AL.: "Spiro asymmetric induction. Synthesis of optically pure .alpha.-hydroxy acid derivatives by alkylation of a chiral glycolate enolate"**
- **LIEBIGS ANNALEN DER CHEMIE, Nr. 8, 8.August 1995, WEINHEIM DE, Seiten 1427-31, XP002021580 H.-J. ALTENBACH ET AL.: "Synthesis of epimeric pure spiro N,S-acetals of L-menthone"**
- **TETRAHEDRON, Bd. 49, Nr. 20, 1993, OXFORD GB, Seiten 4339-54, XP002021581 SK. ASROF ALI ET AL.: "Cycloaddition of 5-substituted 1-pyrrolidine 1-oxide and conversion of the nitrone cycloadducts into cis- and trans-2,5-disubstituted pyrrolidines"**
- **J. ORG. CHEM. (1994), 59(26), 8101-6 CODEN: JOCEAH;ISSN: 0022-3263, 1994, XP002021582 KATAGIRI, NOBUYA ET AL: "A New Synthesis of Highly Functional Nitrones through a Nitrosoketene Intermediate and Their Use for the Stereoselective Synthesis of Amino Acids"**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).

EP 0 854 857 B1

Printed by Jouve, 75001 PARIS (FR)

## Beschreibung

Die Erfindung betrifft ein neues Verfahren zur Herstellung von optisch aktiven Aminosäuren und Aminosäure-Derivaten der allgemeinen Formel I

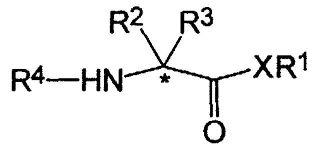

I,

worin

* = Asymmetriezentrum
X = O oder NH
$R^1$ = H, ($C_1$-$C_6$) Alkyl, Benzyl oder ($C_1$-$C_4$) Alkoxycarbonylmethyl und
$R^2$, $R^3$, unabhängig voneinander, H, ($C_1$-$C_6$) Alkyl, das mit Heteroatomen wie N, P, O, S oder Si unterbrochen oder substituiert sein kann, wobei die Heteroatome selbst ein- oder mehrfach mit ($C_1$-$C_3$) Alkyl substituiert sein können, ($C_2$-$C_6$) Alkenyl, ($C_1$-$C_6$) Haloalkyl, Halogen, Aryl, wie Naphthyl oder Phenyl, das ein- oder mehrfach mit ($C_1$-$C_3$) Alkyl, Hydroxy, Halogen oder ($C_1$-$C_3$) Alkoxy substituiert sein kann, Aralkyl wie 2-Naphthylmethyl oder Benzyl, das seinerseits ein- oder mehrfach mit ($C_1$-$C_3$) Alkyl, Hydroxy, Halogen oder ($C_1$-$C_3$) Alkoxy substituiert sein kann, Heteroaralkyl wie N-geschütztes 3-Indolylmethyl und
$R^4$ = H bedeuten,

wobei gilt, wenn $R^2$ = H ist, ist $R^3 \neq$ H.

Proteinogene Aminosäuren sind als Bausteine des Lebens bekannt. Diese α-Aminosäuren können in Peptide eingebaut oder in Aminosäure- bzw. Peptidgemischen, wie Infusionslösungen, verwendet werden.Zunehmend werden jedoch auch nicht proteinogene α-Aminosäuren in Peptide eingebaut, nicht um natürliche Defizite auszugleichen, sondern um gezielt auf Reaktionssequenzen im körperlichen Mechanismus einwirken zu können.

Auch im Bereich der asymmetrischen Synthese stellen insbesondere die verzweigten Aminosäuren wichtige Ausgangsstoffe dar.

Für die genannten Anwendungsgebiete ist es entscheidend, daß die genannten Aminosäuren in möglichst hoher chemischer und optischer Reinheit erhältlich sind.

So ist in Tetrahedron 44 (1988) 5277 ein Verfahren beschrieben, das R- bzw. S-tert.Butyl-methyl-imidazolidinon (R- bzw. S-BMI) in geschützter Form als Glycin-α-Anionenequivalent verwendet. Das Verfahren zur Darstellung des genannten enantiomerenreinen Bausteins basiert zunächst auf der racemischen dreistufigen Synthese. Nach Aminolyse von Glycinmethylester-hydrochlorid mit Monomethylamin in Methanol wird das entstandene Glycinmethylamid mit Pivaldehyd in Methyl-tert-butylether zur Schiff'schen Base kondensiert und anschließend in Ethanol mit einer ethanolischen HCl-Lösung zu rac-BMI-hydrochlorid cyclisiert. Die Enantiomerentrennung erfolgt schließlich mit (R)-Mandelsäure (EP 0 542 099 A2). Das so erhaltene R-bzw. S-BMI kann anschließend alkyliert und zur Aminosäure gespalten werden.

In Aldrichimica Acta 25 (1992) 11 ist ein weiteres Verfahren beschrieben, das auf einem chiralen Oxazinon beruht und das sowohl als Glycin-α-Anionenequivalent (in Form seines BOC-geschützten Derivates) als auch als Glycin-α-Kationequivalent (nach NBS-Bromierung in $CCl_4$) fungieren kann. Als chirale Hilfsfunktion dient erythro-1,2-Diphenylethanolamin, das durch Racematspaltung mit L-Glutaminsäure oder durch asymmetrische Sharpless-Dihydroxylierung erhalten wird. Die Gewinnung der enantiomerenreinen Aminosäuren gelingt oxidativ oder reduktiv. Die Alkylierung des Glycinenolates erfolgt typischerweise in THF durch Zugabe einer starken Base zum Gemisch des Oxazinons und des Elektrophils bei -80 °C. Werden die Reaktionsbedingungen nicht sorgfältig eingehalten, beobachtet man neben unumgesetztem Ausgangsmaterial unerwünschtes Dialkylierungsprodukt. Eine gezielte Zweitalkylierung zu α,α'-substituierten Derivaten läßt sich mit reaktiven Alkylhalogeniden (Allyl-, Benzyl-) ausschließlich bei sehr tiefen Temperaturen (z. B. -80 °C) realisieren. Die komplementäre Glycin-α-Kationroute bedient sich eines α-Bromderivates, das durch NBS-Bromierung in $CCl_4$ erhalten wird. Die instabile Substanz wird direkt als Rohprodukt zur Alkylierung benutzt. In Gegenwart von Zinkchlorid kann eine Bandbreite von Organometallreagenzien eingesetzt werden (Zinn-, Silizium-, Kupfer-, Zinkorganische Verbindungen). Die Kationenroute (α-Haloderivate) liefert Alkyl- und Aryl-Derivate in Ausbeuten zwischen 39 und 82 %, aus denen α-Aminosäuren mit einem ee von 82 % bis 99 % erhältlich sind.

Die beschriebenen Verfahrensvarianten haben die Nachteile, daß sie insbesondere bei sterisch anspruchsvollen Aminosäuren nur moderate Ausbeuten liefern, zum Teil sehr viele Reaktionsschritte benötigen, instabile und nicht lagerfähige Zwischenprodukte bilden sowie das Stereozentrum des chiralen Auxiliars bei der Hydrolyse zerstören und somit eine Recyclierung des Auxiliars unmöglich machen.

Aufgabe der Erfindung ist es demnach, ein Verfahren zur Verfügung zu stellen, das die genannten Nachteile nicht aufweist und zudem ökonomisch verläuft.

Die Aufgabe der Erfindung zur Herstellung von Aminosäuren und Aminosäure-Derivaten der allgemeinen Formel I

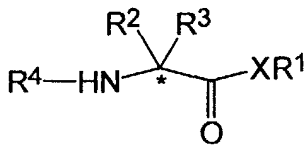

I,

worin

* = Asymmetriezentrum
X = O oder NH
$R^1$ = H, ($C_1$-$C_6$) Alkyl, Benzyl oder ($C_1$-$C_4$) Alkoxycarbonylmethyl und
$R^2$, $R^3$, unabhängig voneinander, H, ($C_1$-$C_6$) Alkyl, das mit Heteroatomen wie N, P, O, S oder Si unterbrochen oder substituiert sein kann, wobei die Heteroatome selbst ein- oder mehrfach mit ($C_1$-$C_3$) Alkyl substituiert sein können, ($C_2$-$C_6$) Alkenyl, ($C_1$-$C_6$) Haloalkyl, Halogen, Aryl, wie Naphthyl oder Phenyl, das ein- oder mehrfach mit ($C_1$-$C_3$) Alkyl, Hydroxy, Halogen oder ($C_1$-$C_3$) Alkoxy substituiert sein kann, Aralkyl wie 2-Naphthylmethyl oder Benzyl, das seinerseits ein- oder mehrfach mit ($C_1$-$C_3$) Alkyl, Hydroxy, Halogen oder ($C_1$-$C_3$) Alkoxy substituiert sein kann, Heteroaralkyl wie N-geschütztes 3-Indolylmethyl und
$R^4$ = H bedeuten,

wobei gilt, wenn $R^2$ = H ist, ist $R^3 \neq$ H,
wird dadurch gelöst, daß man eine allgemeine Verbindung der Formel II

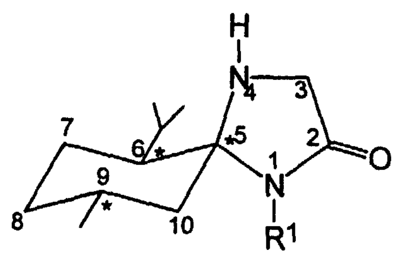

II,

worin *, $R^1$ die oben angegebene Bedeutung besitzen,

a) durch Oxidation in eine Verbindung der allgemeinen Formel III

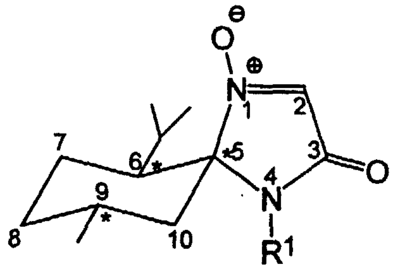

III,

worin *, $R^1$ die oben angegebene Bedeutung besitzt,
überführt und

b) die Verbindungen der allgemeinen Formel III mit einem Nucleophil zu Verbindungen der allgemeinen Formel IV

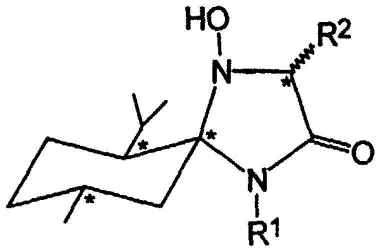

IV,

worin *, $R^1$ und $R^2$ die oben angegebene Bedeutung besitzen, umsetzt und

c) die Verbindungen der allgemeinen Formel IV zu den Verbindungen der allgemeinen Formel V

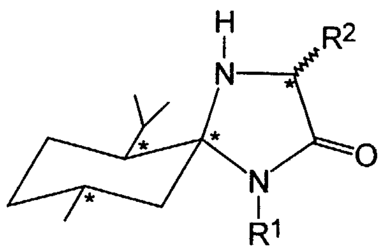

V,

worin *, $R^1$ und $R^2$ die oben angegebene Bedeutung besitzen, reduziert und

d) anschließend die Verbindungen der allgemeinen Formel V zu den L-α-Aminosäuren bzw. L-α-Aminosäure-Derivaten oder D-α-Aminosäuren bzw. D-α-Aminosäure-Derivaten der allgemeinen Formel I oder einem Säureadditionssalz hiervon hydrolysiert oder daß man die Verfahrensschritte a) und b) durchführt und anschließend

e) die Verbindung der allgemeinen Formel IV zu Verbindungen der allgemeinen Formel VI

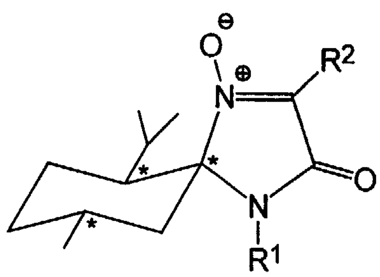

VI,

worin *, $R^1$ und $R^2$ die bereits angegebene Bedeutung besitzen, oxidiert und VI durch Umsetzung mit einem Nucleophil in Verbindungen der allgemeinen Formel VII

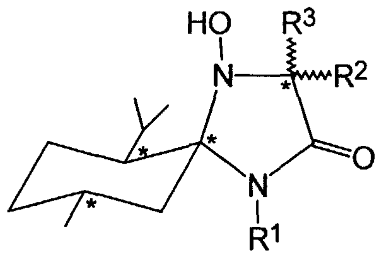

VII,

worin *, $R^1$, $R^2$ und $R^3$ die bereits angegebene Bedeutung besitzen,
überführt und

f) Verbindungen der allgemeinen Formel VII zu Verbindungen der allgemeinen Formel VIII

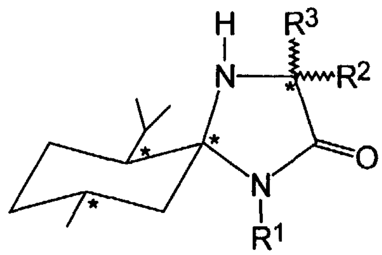

VIII,

worin *, $R^1$, $R^2$ und $R^3$ die oben angegebene Bedeutung besitzen,
reduziert und anschließend

g) zu den α,α-Dialkylaminosäuren bzw. α,α-Dialkylaminosäure-Derivaten der allgemeinen Formel I oder einem Säureadditionssalz hiervon hydrolysiert.

Besonders bevorzugt können nach dem erfindungsgemäßen Verfahren Aminosäuren und Aminosäure-Derivate der allgemeinen Formel I oder ein Säureadditionssalz hiervon

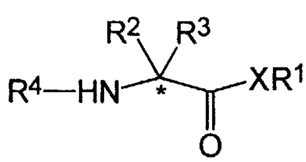

I,

worin

$R^1$ = ($C_1$-$C_4$) Alkyl oder Benzyl,
$R^2$, $R^3$, unabhängig voneinander, H, ($C_1$-$C_6$) Alkyl, das mit Heteroatomen wie N, P, O, S oder Si unterbrochen oder substituiert sein kann, wobei die Heteroatome selbst ein- oder mehrfach mit ($C_1$-$C_3$) Alkyl substituiert sein können, ($C_2$-$C_6$) Alkenyl, ($C_1$-$C_6$) Haloalkyl, Aryl, wie Phenyl, das ggf. ein- oder mehrfach durch Halogen substituiert sein kann, Aralkyl wie Benzyl, das seinerseits ein- oder mehrfach mit ($C_1$-$C_3$) Alkyl, Hydroxy oder ($C_1$-$C_3$) Alkoxy substituiert sein kann, und
$R^4$ = H bedeuten,

erhalten werden,
wobei gilt, wenn X = O ist, kann $R^1$ = H sein, und wenn $R^2$ = H ist, ist $R^3 \neq$ H.

Unter der Bezeichnung "Alkylgruppen" sind sowohl "geradkettige" als auch "verzweigte" Alkylgruppen zu verstehen. Unter der Bezeichnung "geradkettige Alkylgruppe" sind beispielsweise Reste wie Methyl, Ethyl, n-Propyl, n-Butyl, n-Pentyl, n-Hexyl, unter "verzweigter Alkylgruppe" Reste wie beispielsweise Isopropyl, Neopentyl oder tert.-Butyl zu verstehen. Die Bezeichnung Halogen steht für Fluor, Chlor, Brom oder Jod. Die Bezeichnung "Alkoxygruppe" stellt Reste wie beispielsweise Methoxy, Ethoxy, Propoxy, Butoxy, Isopropoxy, Isobutoxy oder Pentoxy dar.

Bei den Verbindungen der Strukturtypen IV, V, VII, VIII, IX, X, XI, XII und XIII entspricht die Nomenklatur dem Strukturtyp II. Bei den Verbindungen des Typs VI ist die Nomenklatur von III anzunehmen.

Das neue Verfahren erlaubt gegenüber den bekannten Verfahren eine neuartige Reaktionsführung, die eine einfachere und schnellere Herstellung der Verbindungen des allgemeinen Typs I ermöglicht. Je nach gewähltem Ausgangsmaterial können gezielt L-$\alpha$-Aminosäuren oder D-$\alpha$-Aminosäuren oder in einem weiteren Reaktionsschritt optisch aktive $\alpha,\alpha$-Dialkylaminosäuren und deren Derivate synthetisiert werden.

So können vorteilhafterweise ausgehend von (-)-Menthol die entsprechenden L-$\alpha$-Aminosäuren bzw. ihre Derivate und ausgehend von dem optischen Antipoden (+)-Menthol die entsprechenden D-$\alpha$-Aminosäuren bzw. ihre Derivate erhalten werden. Die Kennzeichnung der asymmetrischen Kohlenstoffe im Cyclohexanfragment der Verbindungen der allgemeinen Formeln II bis XIII mit * soll deutlich machen, daß es sich hierbei um ein optisch reines Stereozentrum der Konfiguration 6S,9R oder 6R,9S, je nach gewähltem Ausgangsmaterial (-)-Menthol oder (+)-Menthol handelt. Die eindeutige und absolute Konfiguration der weiteren, mit ⁎ gekennzeichneten Asymmetriezentren ergibt sich aus der Wahl der Ausgangsstoffe. So wird in II das Zentrum 5R bzw. 5S eindeutig von 6S,9R bzw. 6R,9S festgelegt. Die Kennzeichnung einer Bindung in den allgemeinen Formeln mit 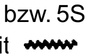 bedeutet, daß es sich um ein Asymmetriezentrum mit eindeutiger und absoluter Konfiguration S bzw. R handelt.

(-)-Menthol bzw. (+)-Menthol wird dabei in an sich literaturbekannter Weise (Houben-Weyl "Methoden der Organischen Chemie", Bd. 7/2a, S. 724) durch Oxidation in das entsprechende (-)-Menthon bzw. (+)-Menthon überführt. Die Darstellung von Verbindungen des allgemeinen Typs II erfolgt ebenfalls in an sich bekannter Weise (Houben-Weyl, 11/2, S. 73ff; EP 0 542 099 A2) beispielsweise durch Umsetzung von Glycinmethylester-Hydrochlorid mit einer alkoholischen Lösung, bevorzugt eine ethanolische Lösung, eines Amins (bei Raumtemperatur und unter Feuchtigkeitsausschluß). Anschließend wird der Suspension (+)- oder (-)-Menthon in Gegenwart von Base zugesetzt und refluxiert. Die Entfernung des Reaktionswassers kann in üblicher Weise erfolgen, bevorzugt wird Molekularsieb (3 Å) verwendet. Nach Beendigung der Reaktion wird das Lösungsmittel entfernt, der Rückstand im Zweiphasensystem Wasser/org. Lösungsmittel aufgenommen, die wäßrige Phase mehrmals mit einem organischen Lösungsmittel extrahiert und die organische Phase anschließend getrocknet. Die Aufreinigung kann durch Destillation oder Umkristallisation erfolgen.

Die Oxidationsreaktion zu Verbindungen des Typs III (Nitron) kann ebenfalls in an sich bekannter Weise (Houben-Weyl, E 14b, S. 1409ff) durchgeführt werden. Dazu wird II beispielsweise in einem organischen Lösungsmittel, wie Methylenchlorid, Aceton oder Methanol, gelöst und mit einem Oxidationsmittel, wie beispielsweise m-Chlorperbenzoesäure, Wasserstoffperoxid in Gegenwart von Natriumwolframat, Selendioxid oder Dimethyldioxiran, umgesetzt. Nach beendeter Reaktion wird das überschüssige Oxidationsmittel zerstört, die organische Phase abgetrennt, die wäßrige Phase mit einem geeigneten Lösungsmittel mehrmals nachgewaschen und die gesammelten organischen Phasen getrocknet. Eine weitere Reinigung kann durch Umkristallisation aus einem organischen Lösungsmittel erfolgen.

Das Nitron III stellt ein bisher unbekanntes Glycin-$\alpha$-Kationenequivalent dar. Im Gegensatz zu den bislang bekannten Synthesen handelt es sich um eine kristalline, lagerfähige Substanz, die hochselektiv mit einer großen Bandbreite von Nucleophilen umgesetzt werden kann. Die alkylierten diastereomerenreinen Derivate IV liegen nach Umkristallisation oder säulenchromatographischer Aufreinigung in Ausbeuten zwischen 39 und 83 % vor.

Die Umsetzung von Verbindungen des Strukturtyps III zu Verbindungen der allgemeinen Formel IV erfolgt unter Feuchtigkeits- und Luftausschluß, z. B. unter Stickstoff- oder Argonatmosphäre. Dazu werden die Verbindungen des Typs III in einem organischen inerten Lösungsmittel, wie DMSO oder einem Ether, beispielsweise Diethylether, Diisopropylether, Dimethoxyethan, THF, Dioxan, oder einem aromatischen Kohlenwasserstoff, wie Toluol, Chlorbenzol, Xylol, oder einem halogenierten Kohlenwasserstoff, wie Methylenchlorid, oder Alkoholen, wie Methanol, Ethanol oder Isopropanol, besonders bevorzugt sind Diethylether oder Toluol, gelöst und auf eine Temperatur zwischen -20 °C bis -80 °C, vorzugsweise -40 °C bis -60 °C, besonders bevorzugt -50 °C, gebracht, bevor 1.0 bis 5.0 Äquivalente, vorzugsweise 2.0 - 3.0 Äquivalente eines Nucleophils (vgl. Lit. P. D. Bailey, J. Clayson, A. N. Boa, Contemp. Org. Syn. 2 (3) 1995, 173, $\alpha$-Cation equivalents of amino acids), typischerweise einer metallorganischen Verbindung, wie beispielsweise einer Organolithium-Verbindung, Organokupfer-Verbindung, Organomagnesium-Verbindung (Grignard-Verbindung), Zink-, Zinn-, Silicium-, Cadmiumorganische Verbindungen oder deprotonierte CH-acide Verbindungen wie Nitromethan, sowie Cyanid, zugegeben werden. Nach Beendigung der Reaktion wird die Reaktionsmischung in üblicher Weise wäßrig, beispielsweise mit eiskalter, halbgesättigter $NH_4Cl$-Lösung, aufgearbeitet. Nach Extraktion der wäßrigen Phase mit einem geeigneten organischen Lösungsmittel wird die organische Phase getrocknet. Die Reinigung des Rohproduktes kann durch Umkristallisation, Destillation oder Säulenchromatographie erfolgen.

Es empfiehlt sich, die bei dieser Reaktion verwendeten Lösungsmittel vorzutrocknen. Prinzipiell können alle denkbaren Organo-Metall-Verbindungen mit III zur Reaktion gebracht werden.

Die Reduktion der Hydroxylamine des Typs IV zu den Aminen der allgemeinen Formel V kann in an sich bekannter Weise erfolgen und analog Houben-Weyl, Bd. 11/1, S. 341ff. Dabei können als Lösungsmittel organische Säuren, wie beispielsweise Essigsäuren, anorganische Säuren, wie beispielsweise HCl, oder organische Lösungsmittel, wie Acetonitril, Alkohole, Ether, Ester, Kohlenwasserstoffe, $CS_2$, in Abhängigkeit des verwendeten Reduktionsverfahrens, zum Einsatz kommen. Die Reaktionstemperatur liegt zwischen -20 °C und +120 °C, vorzugsweise zwischen +20 °C und +60 °C. Die Zugabe des Reduktionsmittels kann in überstöchiometrischen (> 1 Äquivalent bezogen auf IV), stöchiometrischen (1 Äquivalent bezogen auf IV) und in katalytischen Mengen erfolgen.

Zur katalytischen Reduktion können handelsübliche Katalysatoren eingesetzt werden, wie beispielsweise Pd/C, Rh/$Al_2O_3$, Pt/C, Raney-Nickel, Raney-Kobalt, Kupferchromit, Platinoxid, Palladiumhydroxyd. Die hydrogenolytische Reduktion kann bei Normaldruck oder Drucken bis zu 50 atm erfolgen.

Bevorzugt erfolgt die Reduktion in Gegenwart von $CS_2$, Zink oder hydrogenolytisch mit Pd/C, Pt/C oder Ra-Ni. Besonders bevorzugt ist die katalytische Hydrierung mit Pd/C in salzsaurer Lösung bei Normaldruck und Raumtemperatur, eventuell unter Zusatz von Ethanol.

Alternativ erhält man cyclische Iminosäuren, wie beispielsweise cis-4-Hydroxyprolin über eine Cycloaddition an Verbindungen des Typs III. Geeignet zur Cycloaddition sind prinzipiell sowohl elektronenreiche als auch elektronenarme Dipolarophile (siehe Houben-Weyl "Methoden der Organischen Chemie" Bd E16a, S. 327ff, Bd E21c, S. 2953ff, Bd 14b, S. 1523ff und die dort genannten Reaktionsbedingungen).

Bevorzugt gelingt die Umsetzung mit elektronenreichen Dipolarophilen, wie beispielsweise Acroleinacetal in inerten organischen Lösungsmitteln, wie beispielsweise Toluol, Xylol, Chlorbenzol oder Nitrobenzol, und anschließender reduktiver Öffnung des gebildeten Isoxazolidins in an sich bekannter Weise (siehe Houben-Weyl "Methoden der Organischen Chemie" Bd 10/1, S. 1256ff, Comp. Org. Synth. Vol. 8 "Reduction" S. 648ff) zu einer Verbindung des Typs V mit $R^2 = CH_2CH(OH)CH(OR^5)_2$. Anschließend können diese Verbindungen des allgemeinen Typs V (in Schema 1 Typ XVII als spezielle Form des Typs V) durch Hydrolyse entschützt und reduktiv (analog Bull. Chem. Soc. Jpn. Bd 54, S. 3871 (1981)) in an sich bekannter Weise in Verbindungen des Typs XVIII überführt werden, bevor die Verbindungen des Typs XVIII durch Verseifung (gemäß J. Chem. Soc. Chem. Commun. 11, 1291 (1994)) in ein Hydroxyprolin-Derivat überführt werden. Die Umsetzungen verlaufen in guten Ausbeuten und stereoselektiv (s. Schema 1).

Schema 1:

(Es können verschiedene Acroleinacetale eingesetzt werden)

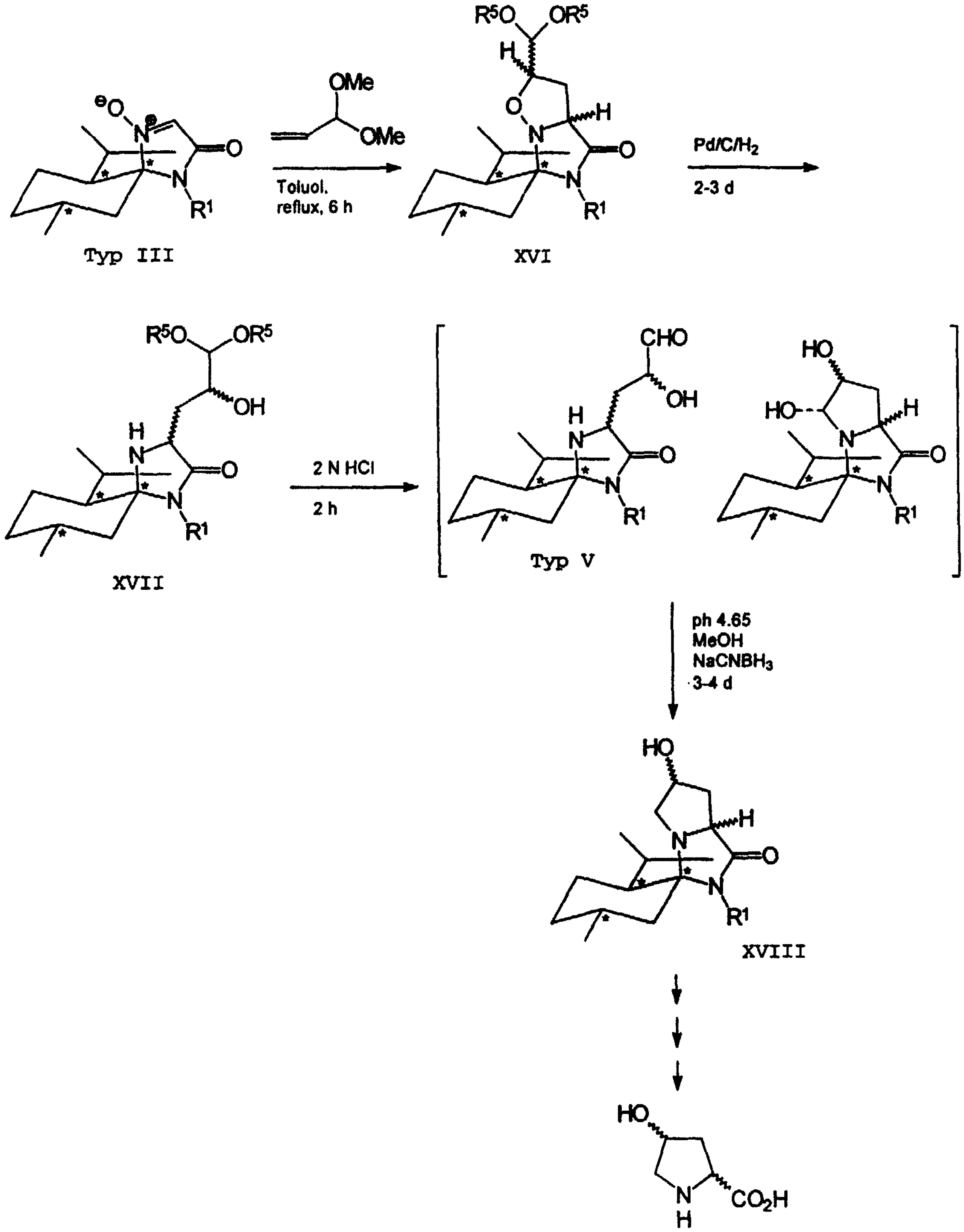


Typ III

XVI

XVII

Typ V

XVIII

cis-Konfiguration

$R^5 = CH_3$, $CH_2CH_3$ oder kann zusammen einen 5-gliedrigen Ring bilden

Eine Synthesevariante stellt die 1,3 dipolare Cycloaddition mit Allylalkohol dar, die im folgenden Schema 2 näher erläutert wird und ebenfalls ein 4-Hydroxyprolin-Derivat liefert.

Schema 2:

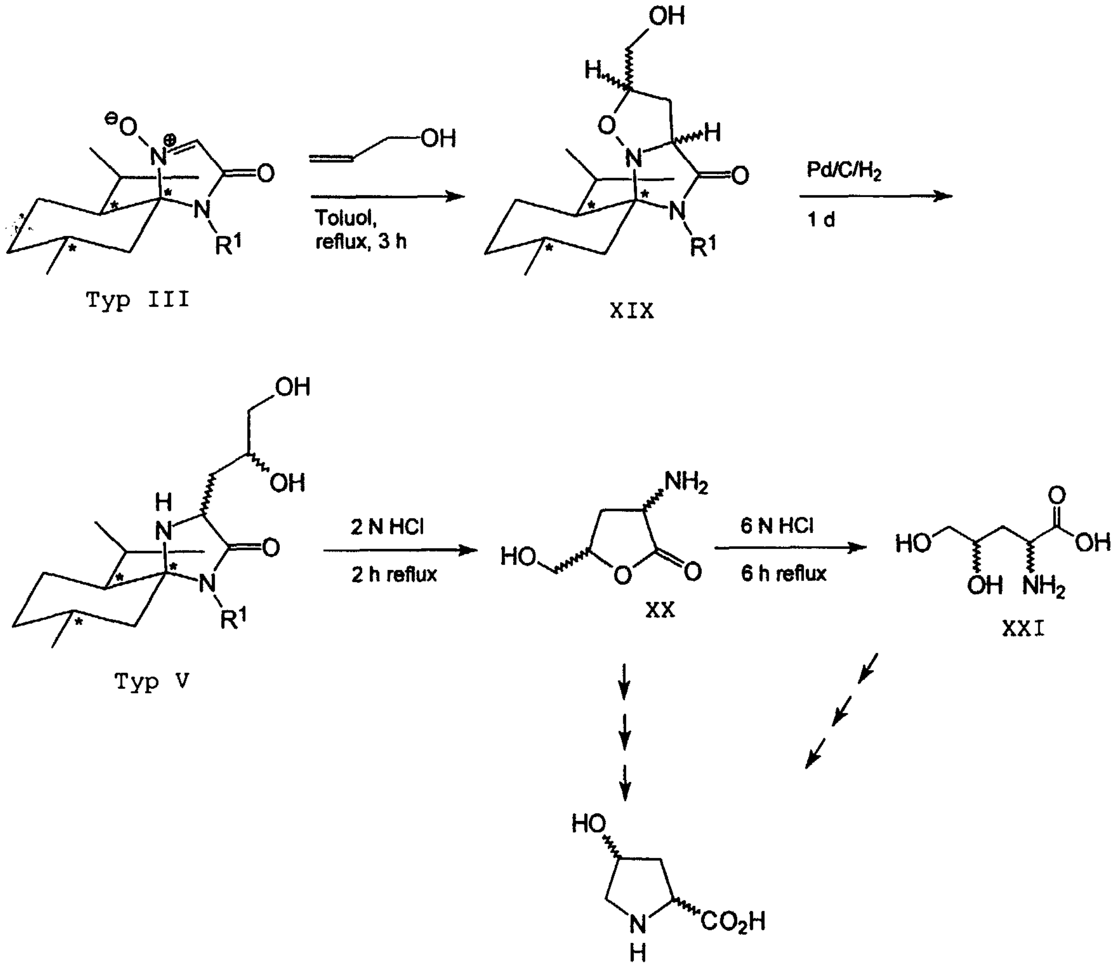


Typ III

XIX

Typ V

XX

XXI

cis-Konfiguration

Die Synthese von cis-4-Hydroxyprolin ausgehend vom Lacton XX oder Verbindung XXI ist bekannt (N. Kurokawa und Y. Ohfune in J. Am. Chem. Soc. 1986, 108, 6041 - 6043).

Die beiden dargestellten Varianten der 1,3 dipolaren Cycloadditionen mit den erfindungsgemäßen Nitronen sind exemplarisch und nicht auf diese beschränkt. Vielmehr wird dadurch die Vielseitigkeit des erfindungsgemäßen Bausteins demonstriert, die problemlose Verwendung und stereoselektive Reaktionsfolge erläutert. $R^1$ hat für beide Reaktionsschemata die oben bereits angegebene Bedeutung.

Die Hydrolyse von Verbindungen der allgemeinen Formel V zu den L- bzw. D-Aminosäuren bzw. L- bzw. D-Aminosäure-Derivaten I kann analog D. Seebach, R. Fitzi, Tetrahedron 44 (1988) 5277 in einem sauren Reaktionsmilieu erfolgen, wie beispielsweise in Gegenwart einer anorganischen Säure, wie HCl, HBr oder $H_2SO_4$, und/oder einem sauren Kationenaustauscher und/oder einer organischen Säure, wie para-TsOH, Camphersulfonsäure, Bromcamphersulfonsäure oder Essigsäure, und/oder einem organischen Lösungsmittel, wie Toluol oder Methanol. Die Reaktion kann bei Temperaturen zwischen 0 °C und 140 °C erfolgen, bei Normaldruck oder auch im Autoklaven. Die Aufarbeitung und Isolation erfolgt in üblicher Weise und analog D. Seebach, R. Fitzi, Tetrahedron 44 (1988) 5277.

Durch geeignete Reaktionsführung in Abhängigkeit von Temperatur und Säurekonzentration kann man entweder optisch aktive ggf. substituierte α-Aminosäureamide oder die entsprechenden α-Aminosäuren erhalten (vgl. D. Seebach, E. Juaristi, D. Müller, Ch. Schickli und Th. Weber, Helv. Chim. Acta, 70 (1987), 237).

Die Verbindungen der allgemeinen Formel I können dabei je nach den gewählten Hydrolysebedingungen auch in Form eines Säureadditionssalzes, wie beispielsweise als HCl-Salz, HBr-Salz, $H_2SO_4$-Salz, para-TsOH-Salz, Camphersulfonsäure-Salz, Bromcamphersulfonsäure-Salz oder Essigsäure-Salz, vorliegen.

Die Umsetzung von Verbindungen der allgemeinen Formel IV zu Verbindungen des Typs VI erfolgt ebenfalls in an sich bekannter Weise analog Houben-Weyl "Methoden der Organischen Chemie", Bd. 10/4, S. 315ff. So wird die

Verbindung IV beispielsweise in einem organischen Lösungsmittel, wie einem halogenierten Kohlenwasserstoff, wie Methylenchlorid, einem aromatischen Kohlenwasserstoff, wie Toluol, einem Ether, wie THF oder in Ethanol + wäßr. $NH_4OH$ oder Eisessig, gelöst und mit einem Oxidationsmittel, wie m-Chlorperbenzoesäure, $O_2$ + Katalysator, $H_2O_2$, tert-Butylhydroperoxid oder Kaliumhexacyanoferrat (III), zur Reaktion gebracht. Nach Beendigung der Reaktion erfolgt übliche Aufarbeitung (vgl. Houben-Weyl "Methoden der Organischen Chemie", Bd. 10/4, S. 315ff).

Alternativ können die Bausteine V durch Umsetzung von Menthon und einem Aminosäureester in Gegenwart einer Aminkomponente, wie beispielsweise Methylamin, Ethylamin, Propylamin, Benzylamin oder Isopropylamin, und einem geeigneten organischen Lösungsmittel, wie beispielsweise einem Alkohol, wie Methanol, Ethanol, Isopropanol, Propanol, n-Butanol, tert.Butanol oder sec.Butanol, erhalten werden. Die so erhaltenen Verbindungen des Typs V können dann durch Oxidation analog der Oxidation des Verbindungstyps II zu Verbindungstyp III in Verbindungen des Typs VI überführt werden.

Alternativ können die Verbindungen des Typs VI auch direkt aus Verbindungen des Typs III erhalten werden. Dazu werden die Verbindungen des Typs III mit einer substituierten Carbonsäure, wie $R^2CO_2H$, wobei $R^2$ die bereits genannte Bedeutung hat, mit Radikalstarter, wie beispielsweise Dibenzoyldiperoxid, Azobisisobutyronitril, $K_2S_2O_8$/ $AgNO_3$ oder $PhI(CF_3CO_2)_2$ (siehe auch Houben-Weyl "Methoden der Organischen Chemie", Bd E19a, Teil 1, S. 140ff) in einem organischen Lösungsmittel, wie einem halogenierten Kohlenwasserstoff, wie Methylenchlorid, Trichlormethan oder Dichlorethan, einem inerten aromatischen Kohlenwasserstoff, wie Benzol, Toluol, Xylol, Chlorbenzol, Nitrobenzol, ggf. unter Erwärmen des Reaktionsgemisches auf 30 °C bis 160 °C, vorzugsweise auf 70 ° - 140 °C, umgesetzt. Die Reaktionskontrolle erfolgt mittels Dünnschicht- oder Gaschromatographie. Die Durchführung der Reaktion findet unter Schutzgasatmosphäre statt, d. h. beispielsweise unter Stickstoff- oder Argonatmosphäre.

Die weitere Umsetzung von Verbindungen des Typs VI zu Verbindungen des Typs VII erfolgt analog der Umsetzung von III zu IV, bei Temperaturen zwischen +80 °C und -50 °C, vorzugsweise bei +25 °C bis -25 °C, besonders bevorzugt bei 0 °C, und bevorzugt mit Toluol als Lösungsmittel.

Die weiteren Umsetzungen von Verbindungen des Typs VII zu VIII und VIII zu I erfolgen analog den Umsetzungen von IV zu V und V zu I (L- bzw. D-Aminosäuren, L- bzw. D-Aminosäure-Derivate) gemäß D. Seebach et al., Liebigs Ann. 1995, 217.

In einer Variante des Verfahrens werden die D-Aminosäuren bzw. D-Aminosäure-Derivate oder die L-Aminosäuren bzw. L-Aminosäure-Derivate der allgemeinen Formel I oder eines Säureadditionssalzes hiervon,

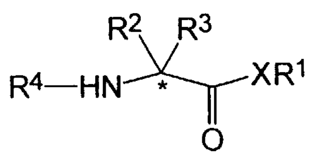

I,

worin

- * = Asymmetriezentrum
- X = O oder NH
- $R^1$ = H, $(C_1-C_6)$ Alkyl, Benzyl oder $(C_1-C_4)$ Alkoxycarbonylmethyl und
- $R^2$, $R^3$, unabhängig voneinander, H, $(C_1-C_6)$ Alkyl, das mit Heteroatomen wie N, P, O, S oder Si unterbrochen oder substituiert sein kann, wobei die Heteroatome selbst ein- oder mehrfach mit $(C_1-C_3)$ Alkyl substituiert sein können, $(C_2-C_6)$ Alkenyl, $(C_1-C_6)$ Haloalkyl, Halogen, Aryl, wie Naphthyl oder Phenyl, das ein- oder mehrfach mit $(C_1-C_3)$ Alkyl, Hydroxy, Halogen oder $(C_1-C_3)$ Alkoxy substituiert sein kann, Aralkyl wie 2-Naphthylmethyl oder Benzyl, das seinerseits ein- oder mehrfach mit $(C_1-C_3)$ Alkyl, Hydroxy, Halogen oder $(C_1-C_3)$ Alkoxy substituiert sein kann, Heteroaralkyl wie N-geschütztes 3-Indolylmethyl und
- $R^4$ = H bedeuten,

wobei gilt, wenn $R^2$ = H ist, ist $R^3 \neq$ H,
dargestellt, indem man

a) die Verbindungen der allgemeinen Formeln IX und X

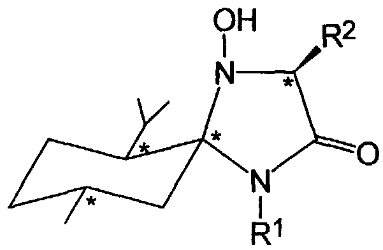

IX

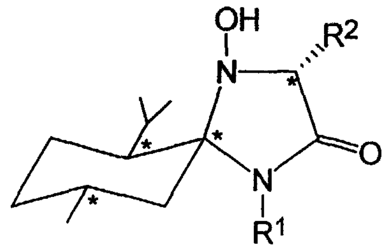

X,

worin *, $R^1$ und $R^2$ die bereits angegebene Bedeutung besitzen,
b) zu Verbindungen der allgemeinen Formel XI

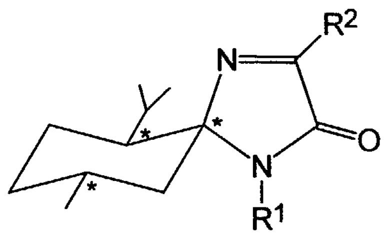

XI,

worin *, $R^1$ und $R^2$ die bereits angegebene Bedeutung besitzen,
dehydratisiert und anschließend die Verbindungen der allgemeinen Formel XI

reduzierend invertiert, so daß aus Verbindungen des Typs IX letztlich Verbindungen des Typs XII und aus Verbindungen des Typs X letztlich Verbindungen des Typs XIII werden (siehe Schema 3).

Schema 3:

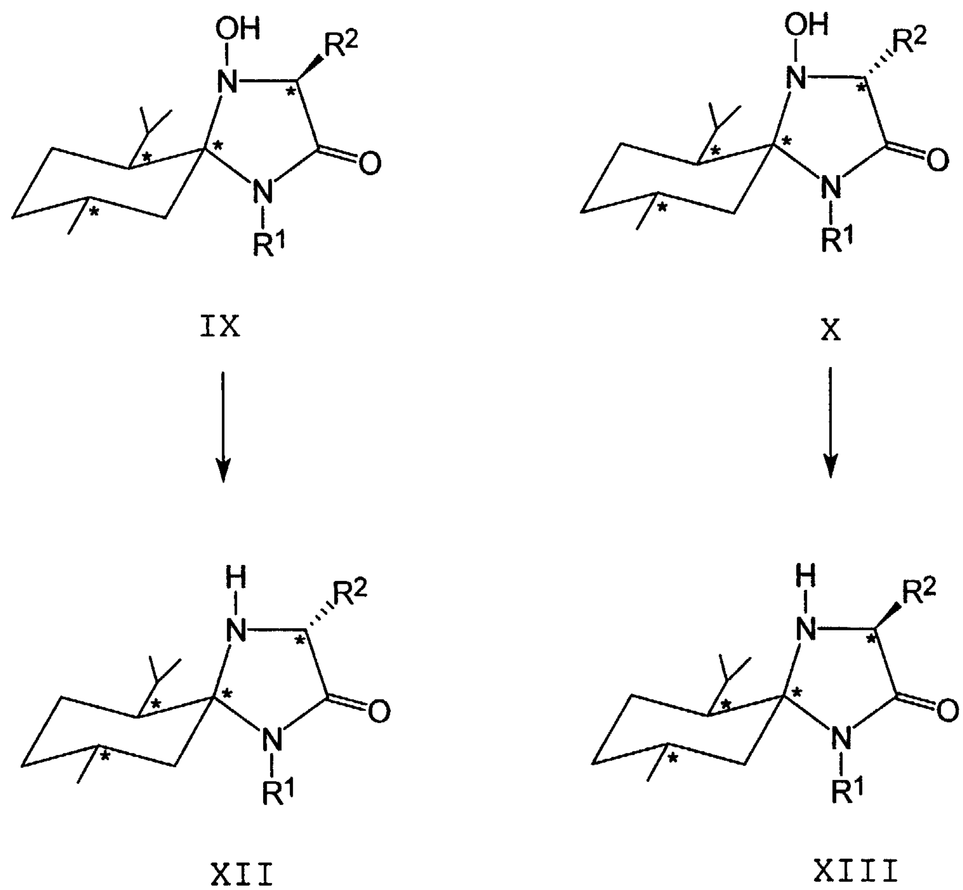

IX X

XII XIII

bevor man die Verbindungen der allgemeinen Formeln XII und XIII zu den Aminosäuren bzw. Aminosäure-Derivaten der allgemeinen Formel I oder einem Säureadditionssalz hiervon hydrolysiert.

Die Dehydratisierung der Verbindungen IX und X zu XI kann in an sich bekannter Weise (Houben-Weyl "Methoden der Organischen Chemie", Bd 10/1, S. 1247, Bd E16a, S. 211ff) erfolgen. So wird beispielsweise die Hydroxylamin-Verbindung des Typs IX oder X in einem organischen Lösungsmittel, wie Methylenchlorid, Pyridin, Ether, in Gegenwart eines wasserentziehenden Mittels, wie N,N'-Carbonyldiimidazol, DCC oder $P_2O_5$, unter Luftausschluß (z. B. $N_2$-Atmosphäre oder Argon-Atmosphäre) bei Temperaturen zwischen 0 °C und 120 °C, vorzugsweise zwischen 20 °C und 30 °C, gerührt und nach Beendigung der Reaktion aufgearbeitet.

Die Verbindungen des allgemeinen Strukturtyps XI können dann unter reduzierenden Bedingungen und Konfigurationsumkehr in die Verbindungen des Typs XII und XIII überführt werden. Die Reduktion kann analog der bereits beschriebenen Reduktion von Verbindungen des Typs IV zu V durchgeführt werden. Bevorzugt ist die katalytische Hydrierung mit $Pd(OH)_2/C$ in Ethanol bei 25 °C und Normaldruck (vgl. auch B. Trost, I. Fleming, Compr. Org. Syn., Bd. 8, "Reductions", Pergamon Press, Oxford 1991).

Die sich anschließende Hydrolyse von den Verbindungen des Typs XII und XIII zu Verbindungen des Typs I erfolgt analog wie für V nach I beschrieben.

Gegenüber einer Deprotonierungs-Reprotonierungssequenz, wie sie an Glycin-α-Anionequivalenten zur Konfigurationsumkehr typischerweise durchgeführt wird (LDA, THF, -78 °C, > 92 % Inversion, D. Seebach, E. Dziadulewicz, L. Behrendt, S. Cantoreggi und R. Fitzi, Liebigs Ann. Chem. 1989, 1215), gelingt vorteilhafterweise eine Dehydratisierungs-Hydrierungssequenz im Verlaufe der Glycin-α-Kationroute des erfindungsgemäßen Verfahrens dagegen bereits bei Raumtemperatur in Ethanol hochselektiv (de > 99 %, GC) ebenfalls in die entsprechende enantiomere Reihe der Aminosäuren (ee = 99 %).

Im folgenden wird das neue Verfahren nochmals an einem Reaktionsschema erläutert:

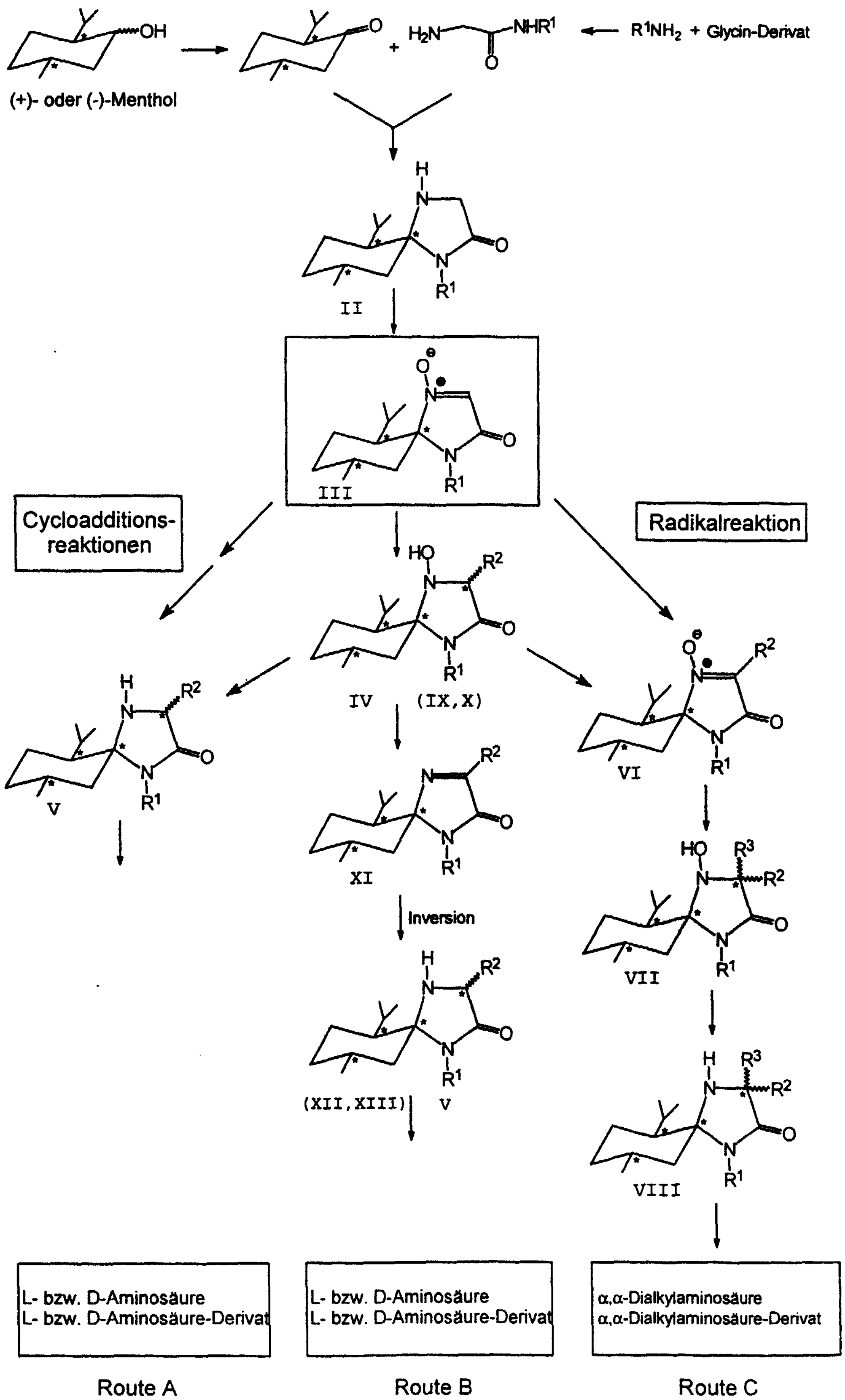
$R^1NH_2$ + Glycin-Derivat
(+)- oder (-)-Menthol
II
III
Cycloadditions-reaktionen
Radikalreaktion
IV (IX,X)
V
VI
XI
Inversion
VII
(XII,XIII) V
VIII
L- bzw. D-Aminosäure
L- bzw. D-Aminosäure-Derivat
L- bzw. D-Aminosäure
L- bzw. D-Aminosäure-Derivat
α,α-Dialkylaminosäure
α,α-Dialkylaminosäure-Derivat
Route A
Route B
Route C

Im erfindungsgemäßen Verfahren können vorteilhafterweise je nach Wahl des entsprechend konfigurierten Menthols als Ausgangsstoff die entsprechenden konfigurierten Aminosäuren bzw. ihre Derivate erhalten werden. So erhält man ausgehend von (-)-Menthol die L-konfigurierte Spezies während man mit (+)-Menthol die entsprechende D-konfigurierte Spezies erhalten kann. Die Aminosäuren bzw. die Aminosäure-Derivate werden dabei in guten chemischen Ausbeuten und hohen stereochemischen Reinheiten mit teilweise > 99 % erhalten.

Bei den Verbindungen der allgemeinen Formel IV handelt es sich um Verbindungen, die einen sehr hohen Enantiomerenüberschuß haben.

Alternativ gelingt es im erfindungsgemäßen Verfahren über die Zwischenstufe XI ausgehend von (-)-Menthol, in die Reihe der D-Aminosäuren bzw. D-Aminosäure-Derivate und ausgehend von (+)-Menthol in die Reihe der L-Aminosäuren bzw. L-Aminosäure-Derivate zu gelangen.

Weiterhin können über die Zwischenstufen VI, VII und VIII α,α-Dialkylaminosäuren bzw. α,α-Dialkylaminosäure-Derivate hergestellt werden.

Ebenfalls vorteilhafterweise ist Menthon wasserdampfflüchtig und kann nach der Freisetzung der Aminosäuren als chirales Auxiliar besonders leicht abgetrennt und recycliert werden.

Ein weiterer Vorteil des Verfahrens ist, daß die Ausgangsstoffe (+)-Menthol als auch (-)-Menthol kommerziell zu günstigen und ähnlichen Preisen problemlos erhältlich ist.

In einer weiteren Variante gelingt es mit dem erfindungsgemäßen Verfahren, N-Hydroxy-Aminosäuren der allgemeinen Formel I

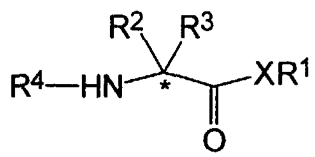 I,

worin

* = Asymmetriezentrum
X = O oder NH
$R^1$ = H, ($C_1$-$C_6$) Alkyl, Benzyl oder ($C_1$-$C_4$) Alkoxycarbonylmethyl und
$R^2$, $R^3$, unabhängig voneinander, H, ($C_1$-$C_6$) Alkyl, das mit Heteroatomen wie N, P, O, S oder Si unterbrochen oder substituiert sein kann, wobei die Heteroatome selbst ein- oder mehrfach mit ($C_1$-$C_3$) Alkyl substituiert sein können, ($C_2$-$C_6$) Alkenyl, ($C_1$-$C_6$) Haloalkyl, Halogen, Aryl, wie Naphthyl oder Phenyl, das ein- oder mehrfach mit ($C_1$-$C_3$) Alkyl, Hydroxy, Halogen oder ($C_1$-$C_3$) Alkoxy substituiert sein kann, Aralkyl wie 2-Naphthylmethyl oder Benzyl, das seinerseits ein- oder mehrfach mit ($C_1$-$C_3$) Alkyl, Hydroxy, Halogen oder ($C_1$-$C_3$) Alkoxy substituiert sein kann, Heteroaralkyl wie N-geschütztes 3-Indolylmethyl und
$R^4$ = OH bedeuten,

wobei gilt, wenn $R^2$ = H ist, ist $R^3 \neq$ H, herzustellen,
indem man Verbindungen der allgemeinen Formel IV

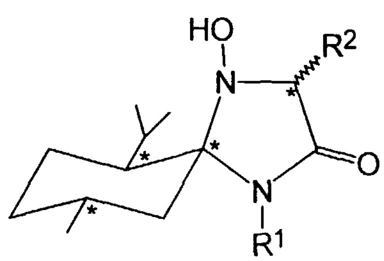 IV,

mit einem metallorganischen Reagenz, ggf. in Gegenwart eines Lösungsmittels, zu Verbindungen der Formel XIV

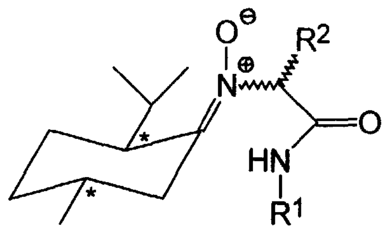

XIV

umsetzt und XIV anschließend, ggf. in Gegenwart eines Lösungsmittels und ggf. in Gegenwart einer Säure, zu Verbindungen des Typs I weiterreagieren läßt.

Das Kettentautomere der allgemeinen Struktur XIV kann gezielt erhalten werden durch Umsetzung von IV mit einer metallorganischen (s. o.) Verbindung. Die Verwendung von Methylmagnesium und die aufgezeigten Reaktionsbedingungen an Verbindungen des Typs IV im folgenden Schema 4 sind beispielhaft. $R^1$ und $R^2$ haben die bereits oben angegebene Bedeutung.

Schema 4

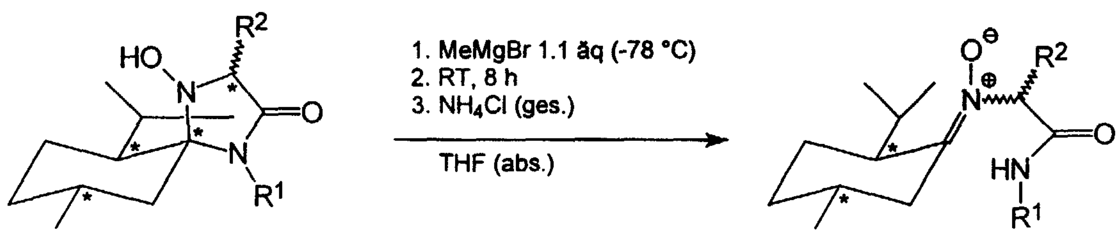


IV

XIV

Unter kontrollierten Bedingungen kann durch Zugabe von 1.0 - 5.0 Äquivalenten, vorzugsweise 1.0 - 1.5 Äquivalenten, eines metallorganischen Reagenzes in einem organischen Lösungsmittel, beispielsweise einem Ether, wie Methyl-tert.Butylether oder THF, zu Verbindungen des Typs IV bei Temperaturen zwischen +30 °C und -100 °C, vorzugsweise 0 °C und -80 °C, eine Ringöffnung von IV zum Kettentautomeren Nitron XIV in guten Ausbeuten erfolgen. Um eine Epimerisierung in α-Stellung zur Amidfunktion zu vermeiden, ist insbesondere darauf zu achten, daß die Lösung des Grignard-Reagenz auch auf -78 °C vorgekühlt ist und die Zutropfgeschwindigkeit nicht zu schnell ist. Die offenkettigen Nitrone entstehen als Gemisch von E/Z-Isomeren. Die Ringöffnungsreaktion verläuft stereoselektiv.

Die Verbindungen des Typs XIV können isoliert werden oder auch in situ anschließend zu den N-Hydroxyaminosäuren hydrolysiert werden. Die Hydrolyse kann gemäß Oppholzer et al., Helv. Chim. Acta 75 (1992), 1965 erfolgen. Bei Verwendung milder Hydrolysebedingungen können auch die entsprechenden Vorstufen der N-Hydroxyaminosäuren, nämlich die N-Hydroxyaminosäureamide x HCl (I, $R^4$ = OH) erhalten werden. Hierzu wird vorzugsweise Salzsäure in Konzentrationen von 0,1 N bis 2 N, besonders bevorzugt 0,5 N, verwendet. Das anschließende Schema 5 verdeutlicht die Reaktion beispielhaft.

Schema 5

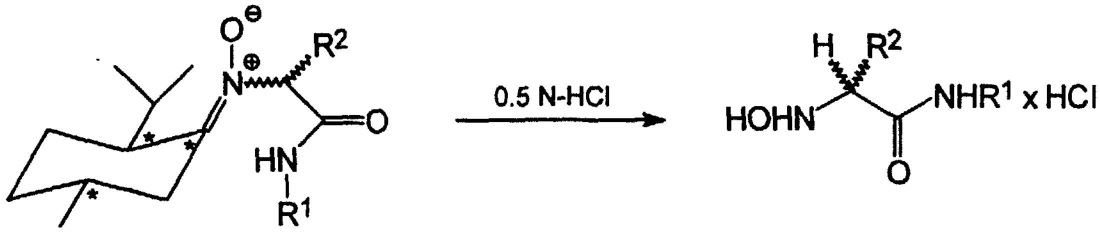


XIV I

Prinzipiell können so, je nach Wahl der Ausgangsbedingungen, N-Hydroxyaminosäure-Derivate in den verschiedenen Konfigurationen (L- bzw. D-) erhalten werden. Anhand der folgenden Ausführungsbeispiele soll die Erfindung näher erläutert werden, ist aber nicht auf diese beschränkt. Die in den Tabellen I bis VIII ausgewiesenen Verbindungen wurden analog den Beispielen 1 - 11, die zum Teil allgemeine Arbeitsvorschriften enthalten, dargestellt.

Beispiele

1. Synthese von (5R,6S,9R)-6-Isopropyl-1,9-dimethyl-1,4-diazaspiro[4.5]decan-2-on (Typ II)

Zu 62.8 g Glycinmethylester-Hydrochlorid (500 mmol) in 160 ml $Ethanol_{abs}$ werden bei Raumtemperatur 156 ml einer käuflichen 8M ethanolischen Methylamin-Lösung gegeben und über Nacht unter Feuchtigkeitsausschluß gerührt. Anschließend wird die Suspension mit 40.5 g Triethylamin (400 mmol) und 61.6 g (-)-Menthon (400 mmol) versetzt und unter Verwendung eines Soxhlet-Aufsatzes, der mit 100 g Molsieb 3Å (Kapazität: 14 %) gefüllt ist, unter Schutzgas (Argon) 18 h unter Rückfluß gekocht. Danach wird das Lösungsmittel entfernt und der Rückstand im Zweiphasensystem Wasser/Diethylether aufgenommen. Nach Abtrennung der organischen Phase wird noch zweimal mit Diethylether extrahiert und über Natriumsulfat getrocknet. Nach Eindampfen der gelben Lösung kann der Rückstand aus Cyclohexan/Diethylether = 8 : 2 umkristallisiert werden. Bei der Destillation der Mutterlauge im Vakuum kann der Anteil der gewünschten Verbindung durch Cyclisierung des Kettentautomeren gesteigert werden. Als erste Fraktion geht zunächst nichtumgesetztes Menthon über (70 °C/1 mbar), gefolgt von einem Gemisch aus gewünschtem Produkt und Kettentautomeren (140 - 150 °C, 1 mbar). Nach Versetzen der Produktfraktion mit Cyclohexan kann weiteres Produkt als kristalliner Feststoff mit einem Schmelzpunkt von 125 °C erhalten werden. Die Ausbeute beträgt 65 %.

2. Synthese von (5S,6S,9R)-6-Isopropyl-4,9-dimethyl-1,4-diazaspiro[4.5]dec-1-en-3-on-1-oxid (Typ III)

22.4 g (5R,6S,9R)-6-Isopropyl-1,9-dimethyl-1,4-diazaspiro[4.5]decan-2-on (Typ II) (100 mmol) in 600 ml $CH_2Cl_2$ werden im Eisbad innerhalb von drei Stunden mit drei Portionen (insgesamt 2.5 Äquivalente, ~70 g) wasserhaltiger m-CPBA (Aldrich) umgesetzt. Die weiße Suspension wird insgesamt 5 h bei 0 °C gerührt (GC-Kontrolle). Nach vollständigem Umsatz wird zur Reduktion überschüssiger Persäure eine 10 %ige $Na_2S_2O_3$-Lösung zugegeben und die Phasen 1 h durch heftiges Rühren gut durchmischt. Danach werden 200 ml gesättigte $NaHCO_3$-Lösung hinzugefügt. Nach Beendigung der $CO_2$-Entwicklung wird die organische Phase abgetrennt und die wäßrige Phase noch zweimal mit $CH_2Cl_2$ extrahiert. Die vereinigten Methylenchlorid-Phasen werden mit ges. $NaHCO_3$-Lösung gewaschen, über Natriumsulfat getrocknet und eingedampft. Das Rohprodukt liegt bereits in sehr reiner Form vor und kann aus Diethylether umkristallisiert werden. (Ausbeute: 94 %, Schmp.: 133.5 °C)

3. Umsetzungen von (5S,6S,9R)-6-Isopropyl-4,9-dimethyl-1,4-diazaspiro[4.5]dec-1-en-3-on-1-oxid (Typ III) mit metallorganischen Reagenzien zu Typ IV

a) 4.76 g (5S,6S,9R)-6-Isopropyl-4,9-dimethyl-1,4-diazaspiro[4.5]dec-1-en-3-on-1-oxid (Typ III) (20 mmol) werden in 400 ml $Diethylether_{abs}$ gelöst und unter Argon auf -50 °C gekühlt. Unter gutem Rühren werden 2.5 Äquivalente einer 1 M Grignard-Lösung oder Lösung eines metallorganischen Reagenzes langsam zugetropft und die entstandene Suspension bei -50 °C nachgerührt (DC-Kontrolle). Nach Erwärmung auf -20 °C wird die Mischung zügig in

250 ml einer eiskalten halbgesättigten $NH_4Cl$-Lösung gegossen, wobei auf gute Durchmischung zu achten ist. Nach Trennung der Phasen wird noch zweimal mit Diethylether extrahiert und die vereinigten organischen Phasen über Natriumsulfat getrocknet. Das Rohprodukt wird durch Säulenchromatographie oder Umkristallisation diastereomerenrein gewonnen.

b) 16.0 g (5S,6R,9R)-6-Isopropyl-4,9-dimethyl-1,4-diazaspiro[4.5]dec-1-en-3-on-1-oxid (Typ III) (67 mmol) werden in 600 ml $Toluol_{abs}$ gelöst. Bei -15 °C tropft man in einer Argonatmosphäre 2.5 Äquivalente einer 1M Grignard-Lösung innerhalb von 2.5 h zu (DC-Kontrolle nach Beendigung der Zugabe). Nach Aufwärmen auf 0 °C gießt man die Suspension unter heftigem Rühren in eine eiskalte halbgesättigte $NH_4Cl$-Lösung. Nach Abtrennen der wässrigen Phase wird diese noch zweimal mit Toluol extrahiert und die vereinigten organischen Phasen über Natriumsulfat getrocknet. Das Produkt wird nach Entfernung des Lösungsmittels im Vakuum durch Säulenchromatographie oder Umkristallisation erhalten.

4. Reduktion der Hydroxylamine (Typ IV) zu den sekundären Aminen (Typ V)

a) Desoxygenierung mit Schwefelkohlenstoff
3 mmol des Hydroxylamins werden in 80 ml $Acetonitril_{abs}$ gelöst und unter Schutzgas mit 18 ml $CS_2$ (300 mmol) bei Raumtemperatur mehrere Tage gerührt. Während dieser Zeit fällt der entstandene Schwefel als flockiger Niederschlag aus. Die Reaktionskontrolle erfolgt gaschromatographisch durch Beobachtung der Zunahme des Produktpeaks (das Edukt zerfällt unter diesen Bedingungen!). Evtl. muß weiteres $CS_2$ zugegeben werden, um die Umsetzung zu vervollständigen. Nach Beendigung der Reaktion wird der gesamte Ansatz einrotiert und in wenig Methanol aufgenommen, wobei der Schwefel als gelbes Pulver zurückbleibt. Nach Filtration und Eindampfen der Lösung kann das Produkt durch Säulenchromatographie an Kieselgel RP-18 gereinigt werden.

b) Reduktion mit Zink
1 mmol der Substanz wird in 8 ml 50 %iger Essigsäure suspendiert und auf 60 °C erwärmt, wobei sich das Edukt vollständig auflöst. 1.70 g Zinkstaub werden unter gutem Rühren in zwei Portionen in die heiße Lösung eingetragen. Nach 30 Minuten wird der Fortschritt der Reaktion gaschromatographisch überprüft und ggf. die Reaktionszeit verlängert. Nach Abbruch der Reaktion wird vom Zink abfiltriert und im Sauren mit Methylenchlorid extrahiert. Die organische Phase wird mit halbgesättigter $NaHCO_3$-Lösung gewaschen, über Natriumsulfat getrocknet und im Vakuum eingedampft.

c) Hydrogenolyse mit Pd/C
76 mg Katalysator (DEGUSSA, E 101 R/W 10 %ig, Wassergehalt 51 %) werden bei 1 atm H2 in 20 ml 1 N HCl bei Raumtemperatur vorhydriert. Danach werden 2 mmol des kristallinen Hydroxylamins über ein Schlenkrohr zugegeben. Anfänglich liegt eine Suspension vor, die sich während des Reaktionsverlaufes über Nacht aufklart. Nach Abbruch der Reaktion wird der flockige Katalysator abfiltriert und die salzsaure Lösung bei 30 °C im Wasserstrahlvakuum eingedampft. Das Produkt liegt i. d. R. bereits sehr sauber vor. Durch Basischstellen mit gesättigter $NaHCO_3$-Lösung und Aufnahme in Essigester können Produktproben gas- und dünnschichtchromatographisch untersucht werden.

5. Dehydratisierung der Hydroxylamine zu Ketiminen (Typ XI)

3.4 mmol der Hydroxylamin-Verbindung werden in 50 ml $CH_2Cl_2$ abs gelöst. Bei Raumtemperatur werden 0.84 g N,N'-Carbonyldiimidazol zugegeben und die klare Lösung in einer Argonatmosphäre 6 h gerührt (Gelbfärbung). Nach Beendigung der Reaktion (GC-Kontrolle) werden 30 ml 0.25 N HCl zugefügt und noch zweimal mit $CH_2Cl_2$ extrahiert. Die vereinigten organischen Phasen werden mit gesättigter $NaHCO_3$-Lösung gewaschen und über Natriumsulfat getrocknet. Die erhaltenen rohen Öle, die bereits eine hohe Reinheit aufweisen, können an Kieselgel chromatographiert werden.

6. Hydrierung der Ketimine zu den sekundären Aminen (Inversion des Stereozentrums)

2 mmol des Ketimins werden in 25 ml $EtOH_{abs}$ gelöst und mit 125 mg $Pd(OH)_2$/C (Aldrich, ~20 %ig) versetzt. Nach Hydrieren über Nacht (1 atm $H_2$, Raumtemperatur) überführt man das sekundäre Amin durch Zusatz von 4 ml 1 N HCl in sein Hydrochlorid. Durch portionsweisen Zusatz von Wasser erhält man aus dem feinstverteilten Katalysator einen flockigen Niederschlag, der sich problemlos abfiltrieren läßt. Nach Eindampfen des Filtrats im Wasserstrahlvakuum bei 30 °C liegt das Produkt als pulveriger Feststoff vor.

Alternativ kann die ethanolische Lösung nach Beendigung der Hydrierung und Zusatz von wenig Dimethylsulfid (150 ml = 2 mmol) durch Celite filtriert und einrotiert werden. Man erhält das freie sekundäre Amin als wachsartige oder ölige Substanz.

7. Reoxidation der Hydroxylamine zum Nitron (Typ VI) (Synthese von (5S,6S,9R)-2-Ethyl-6-isopropyl-4,9-dimethyl-1,4-diazaspiro[4.5]-dec-1-en-3-on-1-oxid)

1.70 g Hydroxylamin (3S,5S,6S,9R)-3-Ethyl-4-hydroxy-6-isopropyl-1,9-dimethyl-1,4-diazaspiro[4.5]-decan-2-on (6.3 mmol) werden in 50 ml $CH_2Cl_2$ gelöst und im Eisbad mit 2.77 g m-CPBA (8.8 mmol, 55 %ig) umgesetzt. Nach 2 h werden 25 ml einer 10 %igen $Na_2S_2O_3$-Lösung und anschließend 40 ml gesättigte $NaHCO_3$-Lösung unter guter Durchmischung zugegeben und nach Beendigung der $CO_2$-Entwicklung ausgeschüttelt. Nach Abtrennung der organischen Phase wird noch zweimal mit $CH_2Cl_2$ extrahiert und die vereinigten organischen Phasen mit gesättigter $NaHCO_3$-Lösung gewaschen. Nach Trocknen über Natriumsulfat und Entfernen des Lösungsmittels im Vakuum liegt ein gelbes Öl vor, das an Kieselgel (Cyclohexan/Essigester = 7 : 3) chromatographiert wird und 67 % an gewünschtem Produkt erhalten. (Drehwert: $[\alpha]_D^{20}$ = +24.5° ($CHCl_3$, c = 0.75))

8. Darstellung der Verbindungen des Typs V aus Aminosäureestern

Zu 0,1 mol Aminosäuremethylester•hydrochlorid gelöst in 50 ml $Ethanol_{abs}$. bzw. n-Butanol werden bei Raumtemperatur 32 ml einer käuflichen 8 M ethanolischen Methylamin-Lösung gegeben. Nach Rühren über Nacht unter Schutzgas wird die Suspension mit 0,1 mol $Triethylamin_{abs.}$ und 0,1 mol Menthon versetzt. Unter Verwendung eines Soxhlet-Aufsatzes, der mit 20 g Molsieb 3 Å (Kapazität: 14 % bei 23 °C) gefüllt ist, wird die Reaktionsmischung 48 h unter Rückfluß gekocht. In der Mitte der Reaktionszeit wird das Molsieb ausgewechselt, um eine möglichst vollständige Umsetzung zu erreichen. Das Lösungsmittel wird entfernt und der gelbe bis braune Rückstand im Zweiphasensystem Diethylether/Wasser aufgenommen. Nach dem Abtrennen der organischen Phase wird noch dreimal mit Diethylether extrahiert, die vereinigte organische Phase über Magnesiumsulfat getrocknet und das Lösungsmittel abgedampft.

9. Umsetzungen der Verbindungen des Typs III zu Verbindungen des Typs VI am Beispiel von 2-tert. Butyl-6-isopropyl-4,9-dimethyl-1,4-diazaspiro[4.5]-dec-1-en-3-on-1-oxid (direkte Route; Radikalreaktion)

Das Lösungsmittelgemisch (12,5 ml $H_2O$, 12,5 ml 1,2-Dichlorethan) wird sorgfältig entgast, die Reaktanden 250 mg (1,05 mmol) Nitron, 321 mg (3,15 mmol; 3 äq) Pivalinsäure, 425 mg (1,58 mmol; 1,5 äq) $K_2S_2O_8$ und 150 mg (1,05 mmol) $AgNO_3$ in der aufgeführten Reihenfolge gelöst und unter Schutzgasatmosphäre am Rückfluß gekocht. Der Reaktionsverlauf wird mittels Dünnschicht- und Gaschromatographie verfolgt und die Reaktion so lange fortgeführt, bis nahezu kein Edukt mehr nachgewiesen werden kann. Gegebenenfalls kann der Reaktionsverlauf zum Ende der Reaktion durch mehrfaches Nachdosieren von Kaliumperoxodisulfat und Silbernitrat bis zu einem maximalen Verhältnis von 10 bzw. 5 Äquivalenten, bezogen auf das eingesetzte Nitron, beschleunigt werden. Zur Aufarbeitung des Ansatzes wird die wässrige Phase abgetrennt und die organische Phase dreimal mit je 20 ml ges. $NaHCO_3$ Lösung ausgeschüttelt. Die vereinigten wässrigen Phasen werden zweimal mit 25 ml Dichlormethan gegengeschüttelt, die organischen Phasen vereinigt, über $MgSO_4$ getrocknet und einrotiert. Das nach vollständiger Entfernung des Lösungsmittels am Hochvakuumrotationsverdampfer erhaltene Rohprodukt wird durch Säulenchromatographie (Kieselgel KG 60, Laufmittel Cyclohexan/Isopropanol 9/1) gereinigt. Man erhält 209 mg (0,71 mmol; 68 %) des gewünschten Produktes als gelbliches Öl.

10. Totalhydrolyse der sekundären Amine zu den freien Aminosäuren

1 mmol des sekundären Amins wird zusammen mit 10 ml 0.75 N HCl, 0.75 ml Eisessig, 1.5 mol Toluol und 10 ml DOWEX 50 W x 8 stark saurem Kationenaustauscher (20 - 50 mesh) in einen Erlenmeyerkolben eingefüllt. Dieser wird mit einem gefetteten Glasstopfen verschlossen und mit einer Stahlklammer gesichert. Man erhitzt das Gemisch für 20 h im Ölbad auf 105 °C (Reaktionskontrolle erfolgt durch Abnahme von wenig Ionenaustauscherharz und Elution mit 10 %iger $NH_3$-Lösung dünnschichtchromatographisch, Laufmittel: Butanol/$H_2O$/HOAc = 3 : 1 : 1). Nach Abbruch der Reaktion überführt man das Ionentauscherharz in eine Glassäule und spült sukzessive mit Ethanol (50 ml) und $H_2O$, bis zur Neutralität. Es wird nun solange mit 10 %iger $NH_3$-Lösung gespült, bis Eluatproben auf Kieselgelplatten eine negative Ninhydrin-Reaktion ergeben. Nach Eindampfen der wässrigen Lösung im Wasserstrahlvakuum wird der feste Rückstand noch zweimal in wenig $H_2O$ gelöst, einrotiert und im Hochvakuum getrocknet. Man erhitzt das weiße Pulver zweimal in $Aceton_{abs}$ zum Sieden und dekantiert nach dem Abkühlen. Die so im Rückstand erhaltene Aminosäure wird auf Enantiomerenreinheit geprüft.

11. Partielle Verseifung von den sekundären Amine des Typs V oder VIII, exemplarisch am Beispiel von (2S)-2-Amino-4-phenylbuttersäure-methylamid

0,80 g (2,4 mmol) (3S,5R,6S,9R)-6-Isopropyl-1,9-dimethyl-3-phenethyl-1,4-diazaspiro-[4.5]decan-2-on werden in 40 ml 5 %iger HCl emulgiert und für 2 h am Rückfluß erhitzt. Nach Abkühlung wird das abgespaltene Menthon mit 2 x 20 ml Ether extrahiert und die wässrige Phase am Rotationsverdampfer einrotiert und an der Hochvakuumapparatur getrocknet. Das polare Rohprodukt wird durch Säulenchromatographie ($CH_2Cl_2$/MeOH = 13/7) gereinigt.

| | |
|---|---|
| Ausbeute: | 0,35 g (75 %) farbloser Kristalle |
| $R_f$: | 0,25 ($CH_2Cl_2$/MeOH = 13/7) |
| Drehwert: | $[\alpha]_D^{20}$ = + 42,51° (c = 1, in MeOH) |

$^1$H-NMR-Spektrum ($CD_3OD$): δ(ppm) = 7,41-7,13 (m, 5 H, Phenylprotonen); 3,94 (dd, 1 H α-ständiges Proton, J = 6,3 Hz); 2,78 (s, 3 H, N-$CH_3$); 2,70 (dd, 2 H, benzylische Protonen, J = 8,4 Hz); 2,13 (m, 2 H, β-ständige Protonen).

Die in den Tabellen abgebildeten Strukturen geben größtenteils die absolute Konfiguration wieder.

Tabelle I: Synthese von Verbindungen des Typs IV

a) ausgehend von (-)-Menthol

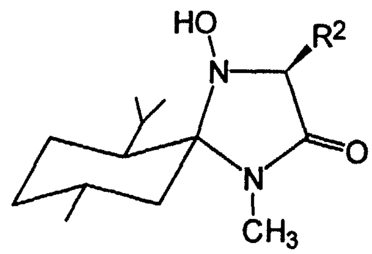

| $R^2$ | Ausbeute in % | Schmp. in °C |
|---|---|---|
| $CH=CH_2$ | 73 | 94.2 |
| $CH(CH_3)_2$ | 39 | 134.1 |
| $C(CH_3)_3$ | 60 [α)] | 171.8 (Zersetzung) |
| $CH_2C_6H_5$ | 71 | 132.6 |
| $CH_3$ | 83 | 175.5 |
| $C_2H_5$ | 77 | 96.8 |

α) mit vorgekühltem tert. Butyllithium als metallorganischem Reagenz und THF als Lösungsmittel

b) ausgehend von (+)-Menthol

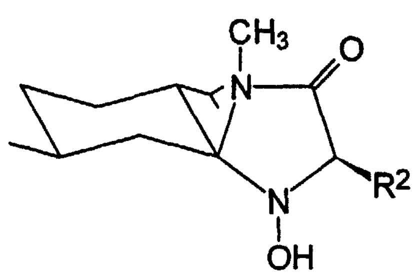

| $R^2$ | Ausbeute in % | Schmp. in °C |
|---|---|---|
| $C_6H_5$ | 26 | 52.7 |
| $p-Br-C_6H_4$ | 11 | Öl |
| $(CH_3)_2CH_2-CH_2$ | 52 | 116.3 |

Tabelle II: Synthese von Verbindungen des Typs V

a) ausgehend von (-)-Menthol

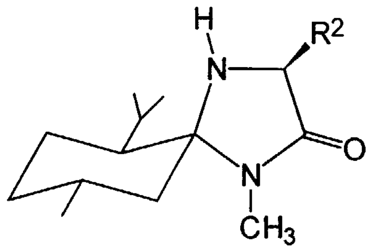

| $R^2$ | **Ausbeute in %** | **Drehwert** $[\alpha]_D^{20}$, $CHCl_3$ |
|---|---|---|
| $CH_3$ | 58[a)]/90[b)]/95[c)] | +8 |
| $CH_2CH_3$ | 72[a)]/98[c)] | -2.2 (c = 3 ) |
| $CH(CH_3)_2$ | 78[a)]/100[b)]/97[c)] | -6 (c = 0.9) |
| $C(CH_3)_3$ | 96[d)] | -6 (c = 0.5) |

a) Umsetzung nach AAV 4a;

b) Umsetzung nach AAV 4b;

c) Umsetzung nach AAV 4c; die Verbindungen fallen als HCl-Salz an;

d) Umsetzung nach AAV 4c und Ethanol-Zusatz. Ausbeute bezogen auf freies sek. Amin nach Basischstellen und Extraktion mit organischem Lösungsmittel.

b) ausgehend von (+)-Menthol

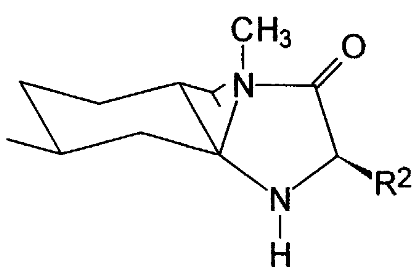

| $R^2$ | **Ausbeute in %** | **Drehwert** $[\alpha]_D^{20}$, c = 0.05, $CHCl_3$ | **Schmelzpunkt in °C** |
|---|---|---|---|
| $C_6H_5$ | 15 | -1.2 | 145.1 |

Tabelle III: Synthese von Verbindungen des Typs XI aus V (ausgehend von (-)-Menthol)

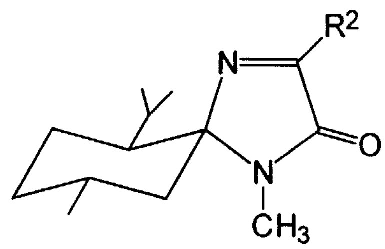

| $R^2$ | **Ausbeute in %** | **Drehwert** $[\alpha]_D^{20}$, **$CHCl_3$** |
|---|---|---|
| $CH=CH_2$ | 80 | +27.5 (c = 1) |
| $CH(CH_3)_2$ | 16 | +5.7 (c = 0.85) |
| $CH_3$ | 93 | +7.8° (c = 3.7) |
| $C_2H_5$ | 63 | +5.7° (c = 1.5) |
| $C(CH_3)_3$ | 64 | α) |

α) $^1$H-NMR (400 MHz, $CDCl_3$): δ = 2.84 (s, 3 H, $CH_3$-15); 2.10 - 0.58: Menthyl-, Isopropyl- und tert-Butylprotonen 27 H, darin: 1.34 (s, 9 H, $C(CH_3)_3$); 0.92 (d, 3 H, $^3J$ = 6.6 Hz, $CH_3$-11): 0.84 (d, 3 H, $^3J$ = 6.9 Hz, $CH_3$-13/14); 0.59 (d, 3H, $^3J$ = 6.8 Hz, $CH_3$-14/13).

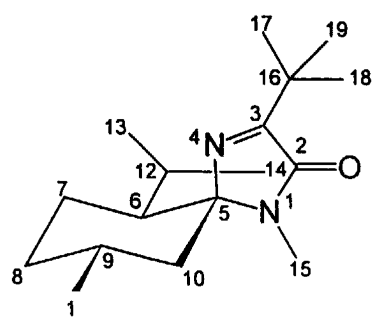

Tabelle IV: Synthese von Verbindungen des Typs VI

1. aus Verbindungen des Typs IV

a) ausgehend von (-)-Menthol

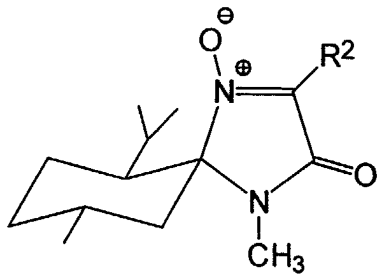

| $R^2$ | **Ausbeute in %** | **Drehwert** $[\alpha]_D^{20}$, **$CHCl_3$** |
|---|---|---|
| $CH_2CH_3$ | 67 | +24.5° (c = 0.75) |
| $CH_3$ | 98 | +94.4° (c = 2.7) |
| $C(CH_3)_3$ | 68 | α) |

α) $^1$H-NMR (400 MHz, $CDCl_3$): δ = 2.87 (s, 3 H, N-$CH_3$); 2.70 (m, 1 H, $H_{ax}$ an C7); 2,2-1,4 (m, 8H, Menthylprotonen, CH (C12); 1.44 (s, 9 H, $CH_3$ t-Butyl); 0.93 (d, 3H, $^3J$ = 6.63 Hz, $CH_3$ (C11); 0.92 (d, 3 H, $^3J$ = 6.88 Hz, $CH_3$ (C13/14); 0.75 (d, 3 H, $^3J$ = 6.78 Hz, $CH_3$ (C13/14)

$^{13}$C-NMR (100.6 MHz, $CDCl_3$): δ = 163,47(s, C-3); 141,03 (s, C-2); 88,43 (s, C-5); 46.51 (d, C-6); 43.41(t, C-10); 34,22 (t, C-8); 33.38 (s, C-16); 27.18 (d, C-9); 26,33 (q, C-17,17",17"); 24,67 (q, C-15); 24.21 (d, C-12); 23.79 (q, C-13/14); 22.34 (q, C-11); 20.11 (t, C-7); 17.05 (q, C-13/14)

b) ausgehend von (+)-Menthol

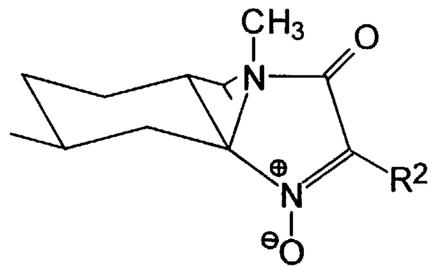

| $R^2$ | Ausbeute in % | Drehwert $[\alpha]_D^{20}$, $CHCl_3$ |
|---|---|---|
| $C_6H_5$ | 96 | -7.85° (c = 0.26) |
| $(CH_3)_2CH_2CH_2$ | 89 | -35.9° (c = 0.93) |

2. aus Verbindungen des Typs III (Radikalreaktion) (siehe Umsetzungsvorschrift 9.)

a) ausgehend von (-)Menthol

α) $R^2$ = Cyclohexyl    Ausbeute: 58 % (Öl)

Als Radikalstarter wurde Bis-(trifluoracetoxy)-iod-benzol verwendet und Benzol als Lösungsmittel. Laufmittel: Dichlormethan/Methanol = 99/1.

$^1H$-NMR (400 MHz, $CDCl_3$): 2.89 (s, 3 H, N-$CH_3$); 2.67 (m, 1 H, $H_{ax}$ an C7); 2,3-1,0 (m, 13 H, Menthylprotonen, CH (C12), Cyclohexylprotonen); 0.92 (d, 3H, $^3J$ = 6.35 Hz, $CH_3$ (C11)); 0.91 (d, 3 H, $^3J$ = 6.82 Hz, $CH_3$ (C13/14)); 0.73 (d, 3 H, $^3J$ = 6.87 Hz, $CH_3$ (C13/14))

β) $R^2$ = $CH(CH_3)_2$    Ausbeute: 48 %

$^1H$-NMR (400 MHz, $CDCl_3$): δ = 3.17 (sept., 1 H, $^3J$ = 7.1 Hz, C-H (C 16)); 2.86 (s, 3 H, N-$CH_3$); 2.64 (m, 1 H, $H_{ax}$ an C7); 2.4-0.7 (m, 24 H, Menthylprotonen, Isopropylprotonen)

γ) $R^2$ = $C(CH_3)_3$ Ausbeute: 68 % (Öl)

$^1H$-NMR (400 MHz, $CDCl_3$): δ = 2.87 (s, 3 H, N-$CH_3$); 2.70 (m, 1 H, $H_{ax}$ an C7); 2.2-1.4 (m, 8 H, Menthylprotonen, CH (C12)); 1.44 (s, 9 H, $CH_3$ t-Butyl); 0.93 (d, 3H, $^3J$ = 6.63 Hz, $CH_3$ (C11)); 0.92 (d, 3 H, $^3J$ = 6.88 Hz, $CH_3$ (C13/14)); 0.75 (d, 3 H, $^3J$ = 6.78 Hz, $CH_3$ (C13/14))

δ) $R^2$ = $C_6H_5$ Ausbeute: 82 % (Öl)

Als Radikalstarter wurde Dibenzoylperoxid verwendet und als Lösungsmittel Benzol. Zur Aufarbeitung wurden 5 ml 2 N HCl zugesetzt. Laufmittel: Dichlormethan/Methanol 99/1.

$^1H$-NMR (400 MHz, $CDCl_3$): δ = 8.77 (quin; 2H, CH (C17/17'); 7,50 (sept, 3 H, CH (C18/18'/18'') C19); 3.01 (s, 3 H, N-$CH_3$); 2.76 (m, 1 H, $H_{ax}$ an C7); 2.4-1.4 (m, 8 H, Menthylprotonen, CH (C12)); 0.97 (d, 3H, $^3J$ = 6.49 Hz, $CH_3$ (C11)); 0.95 (d, 3 H, $^3J$ = 6.82 Hz, $CH_3$ (13/14)); 0.70 (d, 3 H, $^3J$ = 6.87 Hz, $CH_3$ (13/14))

ω) $R^2$ = adamantyl Ausbeute: 52 % (Öl)

Als Radikalstarter wurde Bis-(trifluoracetoxy)-iod-benzol verwendet. Laufmittel: Dichlormethan/Methanol = 99 : 1

$^1H$-NMR (400 MHz, $CDCl_3$): δ = 2.84 (s, 3 H, N-$CH_3$); 2.63 (m, 1 H, $H_{ax}$ an C7); 2.29 (d, 3 H, CH (C18/18'/18'')); 2.2-1.3 (m, 8 H, Menthylprotonen, CH (C12)), 0.92 (d, 3H, $^3J$ =

6.50 Hz, $CH_3$ (C11)); 0.91 (d, 3 H, $^3J$ = 7.15 Hz, $CH_3$ (C13/14)); 0.76 (d, 3 H, $^3J$ = 6.88 Hz, $CH_3$ (C13/14))

Tabelle V: Synthese von Verbindungen des Typs VII

a) ausgehend von (-)-Menthol

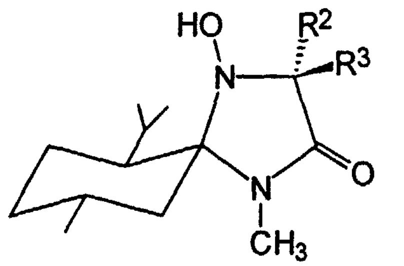

| $R^2$ | $R^3$ | Ausbeute in % | Schmp. in °C |
|---|---|---|---|
| $CH_3$ | $CH_2CH{=}CH_2$ | 54 | 151.2 |
| $CH_3$ | $CH{=}CH_2$ | 84 | 148.5 |
| $CH_2CH_3$ | $CH_3$ | 85 | 148.5 |

b) ausgehend von (+)-Menthol

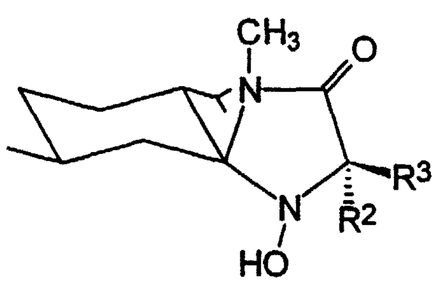

| $R^2$ | $R^3$ | Ausbeute in % | Schmp. in °C |
|---|---|---|---|
| $CH_3$ | $C_6H_5$ | 9 | 182.1 |
| $CH_3$ | p-Br-$C_6H_4$ | 13 | 153.3 |
| $(CH_3)_2CH_2CH_2$ | $CH_3$ | 69 | 201.4 |
| $CH_2CH_3$ | $CH_3$ | 56 | 142.1 |

Tabelle VI: Synthese von Verbindungen des Typs VIII (ausgehend von (+)-Menthol)

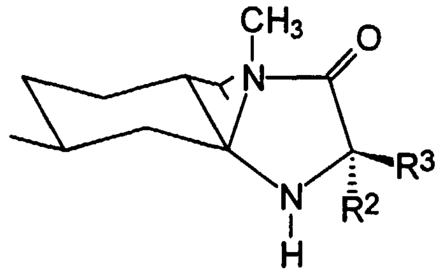

| $R^2$ | $R^3$ | Ausbeute in % | Schmp. in °C |
|---|---|---|---|
| $CH_3$ | $C_6H_5$ | 74 | - |
| $(CH_3)_2CH_2CH_2$ | $CH_3$ | 91 | 122.0 |
| $CH_2CH_3$ | $CH_3$ | 30 | 111.2 |

Tabelle VII: Synthese von Verbindungen des Typs X aus IX über XI (ausgehend von (-)-Menthol)

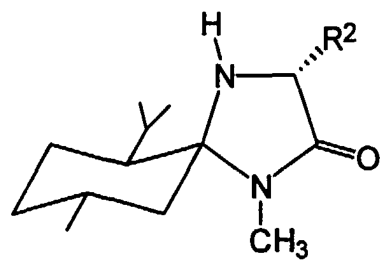

| $R^2$ | Ausbeute in % | Drehwert $[\alpha]_D^{20}$, $CHCl_3$ |
|---|---|---|
| $CH_3$ | 96 | - |
| $CH_2CH_3$ | 85 | -2.3 |

Tabelle VIII:

| Synthese von Verbindungen des Typs V mit Hilfe von Aminosäureestern und (+)-Menthon (Die Umsetzungen erfolgen analog den Umsetzungen zu Verbindungstyp II) | | |
|---|---|---|
| Aminosäure | Lösungsmittel | Ausbeute |
| L-Alanin | EtOH | 71 % |
| L-Methionin | EtOH | 71 % |
| L-Methylcystein | EtOH | 75 % |

Die Umsetzung von (-)-Menthon mit L-Alanin liefert in n-Butanol eine Ausbeute von 70 % an Verbindungstyp V. ×

Tabelle 9:

| Hydrolyse zu den Aminosäuren | | |
|---|---|---|
| Route A | | |
| Aminosäure | Ausbeute in % | ee[a)] in % |
| L-Alanin | 75 | >99.9 |
| L-Aminobuttersäure | 58 | >99 |
| L-Valin | 36 | >99 |
| L-Phenylalanin | 70 | >99 |
| L-Vinylglycin | 29 | 20 |
| L-tert-Leucin×HCl×X$H_2O$ | ~90 | >99 |
| a) Die Enantiomerenreinheit wurde nach Derivatisierung über HPLC bestimmt. | | |
| Route B | | |
| Aminosäure | Ausbeute in % | ee in % |
| D-Alanin | 73 | 99.8 |
| D-Aminobuttersäure | 91 | 99.3 |

12. Darstellung von cis-4-Hydroxyprolin-Derivaten durch Cycloaddition am Beispiel cis-4-Hydroxyprolin

1) gemäß Schema 1

a) Aufbau des Isoxazolidins XVI mit Acroleindimethylacetal

500 mg (2.10 mmol) (5S,6S,9R)-6-Isopropyl-4,9-dimethyl-1,4-diazaspiro[4.5]dec-1-en-3-on-1-oxid und 856 mg (8.40 mmol) Acroleindimethylacetal werden in Toluol gelöst und für 18 h unter Argonatmosphäre am Rückfluß gekocht. Der Reaktionsverlauf wird mittels Dünnschichtchromatographie verfolgt und ggf. die Reaktionszeit verlängert, bis kein Edukt mehr vorhanden ist, da die chromatographische Trennung von Edukt und Produkt problematisch ist. Nicht umgesetztes Acroleindimethylacetal wird zusammen mit dem Lösungsmittel am Rotationsverdampfer und nachfolgend an der Hochvakuumapparatur entfernt. Das hellgelbe ölige und unter Vakuum stark schäumende hochviskose Rohprodukt kann für weitere Umsetzungen direkt weiterverwendet werden. Zu Analysezwecken wird es durch Säulenchromatographie (Kieselgel KG 60, $CH_2Cl_2$/MeOH 97/3) gereinigt. Man erhält 607.7 mg (1.78 mmol, 85 %) des Cycloadduktes.

$^1$H-NMR (400 MHz, $CDCl_3$): $\delta$ = 4.29 (d, 1 H, $^3J$ = 6.01 Hz, CH (C18)); 3.94 (d, 1 H, $^3J$ = 8.96 Hz, CH (C2)); 3.86 (m, 1 H, CH (C17)); 3.44 (s, 1 H, $OCH_3$ (C19)); 3.41 (s, 1 H, $OCH_3$ (C19')); 2.74 (s, 3 H, N-$CH_3$); 2.67 (okt., 1 H, CH (C16)); 2.39 (sext., 1 H, CH (C16)); 2.1-1.1 (m, 8 H, Menthylprotonen, CH (C12)); 0.97 (d, 3 H, $^3J$ = 6.63 Hz, $CH_3$ (C11)); 0.88 (d, 3 H, $^3J$ = 6.93 Hz, $CH_3$ (C13/14)); 0.86 (d, 3 H, $^3J$ = 6.75 Hz, $CH_3$ (C13/14))

Bei Verwendung von Acroleindiethylacetal bzw. des 2-Vinyl-1,3-dioxolans als 1,3-Dipolarophil werden 87 % bzw. 81 % der zu XVI analogen Isoxazolidinderivate an Ausbeute isoliert.

b) Ringöffnung zu der Verbindung des Typs XVII

In einem Dreihalskolben wird der Hydrierungskatalysator (100 mg Palladium auf Aktivkohle) vorgelegt, der Kolben gründlich mit Argon gespült und das Lösungsmittel zugegeben. Der Katalysator wird vorhydriert und über ein Septum 400 mg (1.17 mmol) des Cycloaddukts gelöst in 25 ml abs. Methanol zugetropft. Es wird mehrfach Vakuum angelegt und die Apparatur mit Wasserstoff gespült. Die Zufuhr des zur Reaktion benötigten Wasserstoffs erfolgt über Gasballontechnik. Man läßt ca. 1 Woche intensiv rühren, ersetzt alle 24 h den Wasserstoffvorrat und fügt nach 3 Tagen über ein Schlenkrohr noch einmal die gleiche Menge neuen Katalysator zu.

Der Katalysator wird über Kieselgur abgesaugt und das Filtrat am Rotationsverdampfer und an der Hochvakuumapparatur vom Lösungsmittel befreit. Das Rohprodukt wird durch Säulenchromatographie (Kieselgel KG60, Laufmittel: $CH_2Cl_2$/MeOH 97/3) von Restmengen nicht umgesetzten Cycloadduktes gereinigt. Die Ausbeute des farblosen zähflüssigen Öls beträgt 293 mg (0.86 mmol, 73 %).

$^1$H-NMR (400 MHz, $CDCl_3$): δ = 4.70 (s, 1 H, NH-Amin); 4.26 (d, 1 H, $^3J$ = 5.11 Hz, CH (C18)); 3.89 (okt. 1 H, CH (C17)); 3.76 (t, 1 H, $^3J$ = 6.17, CH (C2)); 3.48 (s, 6 H $OCH_3$ (C19/19')); 2.79 (s, 3 H, N-$CH_3$); 2.2-1.5 (m, 11 H, 8 Menthylprotonen, CH (C12)), $CH_2$ (C16)); 0.94 (d, 3 H, $^3J$ = 6.46 Hz, CH3 (C11)); 0.91 (d, 6 H, $^3J$ = 6.86 Hz, $CH_3$ (C13/14))

Die entsprechend ringgeöffneten Produkte ausgehend von Acroleindiethylacetal und 2-Vinyl-1,3-dioxolan als Dienophil wurden in 60 % bzw. 54 % Ausbeute isoliert.

c) Synthese von (2S,5S,7S)-7-Hydroxy-3-methyl-4-oxo-1,3-diaza-bicyclo[3.3.0]-octan-2-[(2S,5R)-2-isopropyl-5-methyl]-spirocyclohexan (Verbindung XVIII)

1.4 g (4.02 mmol) des Aminoalkohols Typ XVII werden in 75 ml 1 N Salzsäure suspendiert und für 2.5 h gerührt. Dabei löst sich der Prolinbaustein langsam und die Trübung des Gemisches verschwindet. Das Lösungsmittel wird bei Raumtemperatur an der Hochvakuumapparatur abgezogen, der Rückstand in 50 ml pH 4.62 Puffer (Essigsäure/Acetatpuffer, Fa. Riedel de Haen) aufgenommen und portionsweise insgesamt 1.26 mg (20.1 mmol) Natriumcyanoborhydrid hinzugegeben. Man läßt für insgesamt 5 Tage bei RT rühren und dosiert nach 2 Tagen noch einmal 0.63 g (10 mmol) $NaBH_3CN$ nach. Zur Aufarbeitung wird die Reaktionslösung mit verd. HCl-Lösung vorsichtig angesäuert und fünfmal mit je 30 ml Dichlormethan extrahiert. Die org. Phase wird über $MgSO_4$ getrocknet, das Lösungsmittel abrotiert und an der HV getrocknet. Man isoliert 879 mg (3.13 mmol, 78 %) einer farblosen teilkristallinen Substanz, die dem gewünschten Produkt entspricht. Eine vollständige Reinigung des Rohproduktes erhält man durch Säulenchromatographie (Kieselgel KG60, Laufmittel: Cyclohexan/Isopropanol 8/2).

$R_f$(Produkt): 0.10 ($CH_2Cl_2$/MeOH 97/3)

$^1$H-NMR (400 MHz, $CDCl_3$): δ = 4.33 (t, 1 H, $^3J$ = 4.80 Hz, CH (C17)); 3.72 (q, 1 H, $^3J$ = 4.53 Hz, $^3J$ = 8.98 Hz, CH (C2)); 3.16 (q, 1 H, $^3J$ = 10.50 Hz, $^3J$ = 4.30 Hz, CH (C18)); 3.08 (q, 1 H, $^3J$ = 10.46 Hz, $^3J$ = 4.83 Hz, CH (C18)); 2.74 (s, 3 H, N-$CH_3$); 2.28-1.27 (m, 11 H, Menthylprotonen, CH (C12)), $CH_2$ (C16)); 0.96 (d, 3 H, $^3J$ = 6.21 Hz, $CH_3$ (C11)); 0.89 (d, 3 H, $^3J$ = 6.69 Hz, $CH_3$ (C13/14)); 0.84 (d, 3 H, $^3J$ = 6.80 Hz, $CH_3$ (C13/14))

Weitere Umsetzungen, wie in J. Chem. Soc. Chem. Commune (11) 1291 (1994) beschrieben, führen zu L-cis-4-Hydroxyprolin.

2) gemäß Schema 2

Synthese von (2S,5S,7S)-7-Hydroxymethyl-3-methyl-4-oxo-8-oxa-1,3-diazabicyclo[3.3.0]octan-2-[(2S,5R)-2-isopropyl-5-methyl]-spirocyclohexan (XIX)

(5S,6S,9R)-6-Isopropyl-4,9-dimethyl-1,4-diazaspiro[4.5]dec-1-en-3-on-1-oxid (15.78 mmol, 4 g) und Allylalkohol (50.22 mmol, 2.92 g) werden in 30 ml abs. Toluol unter Argon 5 h unter Rückfluß erhitzt. Nach Abziehen des Lösungsmittels am Rotationsverdampfer und anschließend am Hochvakuum wird das Rohprodukt aus Essigester umkristallisiert. Man erhält XIX (10.66 mmol, 3.16 g) als farblose Kristalle in 64 %iger Ausbeute.

Smp.: 133.5 °C
$[\alpha]_D^{21}$ = +69.8 (c = 1.0, Chloroform)
$R_f$-Wert: 0.22 (Methylenchlorid/Methanol = 96.5:3.5)

Synthese von (3S,5R,6S,9R)-3-[(2S)-2,3-Dihydroxypropyl]-6-isopropyl-1,9-dimethyl-2-oxo-1,4-diazaspiro[4.5]decan (Typ V)

Verbindungstyp XIX (4.35 mmol, 1.29 g) in 40 ml abs. Methanol wird in Gegenwart von 0.4 g Pd/C (10 %) unter Wasserstoffatmosphäre bei Raumtemperatur umgesetzt. Nach 8 h wird die Reaktionslösung über Celite abfiltriert, der Katalysator mit Methanol gewaschen und das Lösungsmittel am Rotationsverdampfer entfernt. Trocknung am Hochvakuum liefert 1.29 g (99 %) farbloses Öl.
$[\alpha]_D^{21}$ = +14.2 (c = 1.0, Chloroform)

Synthese von (3S,5S)-3-Amino-5-hydroxymethyldihydro-2-furanon (XX)

3.62 mmol (1.08 g) (3S,5R,6S,9R)-3-[(2S)-2,3-Dihydroxypropyl]-6-isopropyl-1,9-dimethyl-2-oxo-1,4-diazaspiro[4.5]decan werden in 20 ml 5 %iger HCl 2 h unter Rückfluß erhitzt. Nach Extraktion mit 2 x 30 ml Diethylether wird die wäßrige Phase evakuiert. Das Rohprodukt wird durch Umkristallisation aus Ethanol und Wasser gereinigt und liefert farblose Kristalle in 74 %iger Ausbeute.

Smp.: 230.0 °C (Zersetzung)
$[\alpha]_D^{21}$ = +27.1 (c = 1.0, Chloroform)

Synthese von (2S,4S)-4,5-Dihydroxynorvalin (XXI)

(3S,5S)-3-Amino-5-hydroxymethyldihydro-2-furanon XX (4.8 mmol, 0.80 g) wird in 40 ml 6 N Salzsäure 8 h unter Rückfluß erhitzt. Die Reaktionslösung wird evakuiert, der Rückstand auf eine stark säure Ionenaustauschersäule gegeben, die anschließend mit Wasser neutral gewaschen wird. Danach wird die Aminosäure XXI mit 1.5 N Ammoniaklösung eluiert. Nach Abziehen des Eluierungsmittels wird das Rohprodukt aus Wasser und Ethanol umkristallisiert. Man erhält 365 mg (51 %) der farblosen Zielverbindung vom Schmelzpunkt 154 °C.
$[\alpha]_D^{21}$ = -23.9 (c = 1.0, Wasser)
Weitere Umsetzungen analog J. Am. Chem. Soc. 1986, 108, 6041 führen zum L-cis-4-Hydroxyprolin

13. Synthese von N-Hydroxy-Aminosäuren und Derivate (Allgemeine Arbeitsvorschrift)

Die Synthese erfolgt über die Kettentautomeren der Verbindungen der allgemeinen Formel IV

1) Allgemeine Darstellung des Kettentautomeren XIV (Ringöffnungsreaktion)
1.86 mmol des Verbindungstyps IV werden in 25 ml absolutem THF gelöst und unter Argon auf -78 °C gekühlt. Unter gutem Rühren werden 1.1 Äquivalente einer 3 M Methyl-Grignard-Lösung langsam zugetropft. Die entstandene Suspension wird auf 20 °C erwärmt und nachgerührt (DC-Kontrolle). Anschließend wird die Mischung mit 50 ml einer halbgesättigten $NH_4Cl$-Lösung versetzt. Nach der Trennung der organischen Phase wird noch zweimal mit Diethylether extrahiert und die vereinigten organischen Phasen über Natriumsulfat getrocknet. Die E/Z-Isomere werden durch Säulenchromatographie diastereomerenrein erhalten.

a) Synthese von (1S,2'S,5'R)-1-(N-Methyl-1-carbamoyl)propyl-(2'-isopropyl-5'-methyl)-cyclohexyliden-amin-N-oxid (Typ XIV)
Nach der allgemeinen Arbeitsvorschrift werden 500 mg (0.59 mmol) des Verbindungstyps IV mit $R^1 = CH_3$ und $R^2 = CH_2CH_3$ in 25 ml $THF_{abs.}$ gelöst und mit 0.68 ml einer 3 M Methylmagnesiumbromid-Lösung umgesetzt. Nach Säulenchromatographie in $CH_2Cl_2/MeOH/Et_3N$ (96/3/1) liegen die E/Z-Isomere als farbloser Feststoff vor.

Ausbeute: 81 %.

$R_f$: 0.2/0.27 ($CH_2Cl_2$/MeOH = 95/5)

$^1$H-NMR (400 MHz, $CDCl_3$,): δ = 8.26 (s, breit, 1 H, CON$\underline{H}$$CH_3$); 4.76 (dd, 1 H, $^3J_A$ = 5.7 Hz, $^3J_B$ = 8.6 Hz, CH-3); 3.51 (dm, 1 H, $^3J$ = 13.92 Hz, $CH_e$-10); 2.76 (d, 3 H, $^3J$ = 4.9 Hz, CONHC$\underline{H}_3$); 0.98 (t, 3 H, $^3J$ = 7.4 Hz, $CH_3$-17); 0.95 (d, 2 H, $^3J$ = 7.1 Hz, $CH_3$-11/13/14); 0.92 (d, 3 H, $^3J$ = 7.1 Hz, $CH_3$-11/13/14); 0.82 (d, 3 H, $^3J$ = 6.7 Hz, $CH_3$-11/13/14).

b) Synthese von (1S,2'S,5'R)-1-(N-Methyl-1-carbamoyl)-2,2-dimethyl-propyl-(2'-isopropyl-5'-methyl)-cyclohexyliden-amin-N-oxid (Typ XIV)

Nach der allgemeinen Arbeitsvorschrift werden 220 mg (0.74 mmol) des Verbindungstyps IV mit $R^1 = CH_3$ und $R^2 = C(CH_3)_3$ in 15 ml THF gelöst und mit 0.27 ml einer 3 M Methylmagnesiumbromid-Lösung umgesetzt. Nach Säulenchromatographie in $CH_2Cl_2$/MeOH/$Et_3N$ (96/3/1) liegen die E/Z-Isomere als farbloses Öl vor.

Ausbeute: 62 %

$R_f$: 0.48 ($CH_2Cl_2$/MeOH = 95/5), UV

$^1$H-NMR (400 MHz, $CDCl_3$): E/Z-Isomere: $\delta$ = 8.8/8.5 (s, breit, 1 H, CONHC$\underline{H}_3$); 4.58/4.54 (s, 1 H, CH-3); 3.47 (dm, $CH_e$-10); 2.74/2.71 (d, 3 H, CONHC$\underline{H}_3$, $^3J$ = 4.9 Hz); 2.46-0.79 Menthyl- und tert.Butylprotonen: 26 H, darin: 1.16/1.15 (s, 9 H, $CH_3$-17/18/19); 0.97 (d, 3 H, $CH_3$-11/13/14, $^3J$ = 6.5 Hz); 0.89 (d, 3 H, $^3J$ = 7.1 Hz, $CH_3$-11/13/14); 0.83 (d, 3 H, $^3J$ = 6.8 Hz, $CH_3$-11/13/14); 0.80 (d, 3 H, $^3J$ = 6.7 Hz, $CH_3$-11/13/14) .
Auf analogem Wege können weitere Derivate hergestellt werden:

c) (1S,2'S,5'R)-1-(N-Methyl-1-carbamoyl)-ethyl-(2'-isopropyl-5'-methyl)-cyclohexyliden-amin-N-oxid

Ausbeute: 73 %; E/Z-Verhältnis: 6/1

d) (1S,2'S,5'R)-1-(N-Methyl-1-carbamoyl)-2-methyl-propyl-(2'-isopropyl-5'-methyl)-cyclohexyliden-amin-N-oxid

Ausbeute: 64 %; E/Z-Verhältnis: 3/1

Anmerkung: Die Nomenklatur für das Kettentautomere XIV erfolgt gemäß Houben-Weyl "Methoden der Organischen Chemie", Bd E14b, Teil 2.

2) Hydrolyse zu den N-Hydroxy-aminosäuren und Derivate

a) N-Hydroxy-L-Alaninmethylamid-hydrochlorid

254 mg (1 mmol) (Ξ)-(1S,2'S,5'R)-1-(N-Methyl-1-carbamoyl)-ethyl- (2'-isopropyl-5'-methyl) -cyclohexyliden-amin-N-oxid werden in 4 ml 0.5 N HCl gelöst und 2 h stehengelassen. Beim Eindampfen im Wasserstrahlvakuum wird auch das freigewordene Menthon ausgetrieben. Man erhält 170 mg einer gelblichen zähen Masse, die sich NMR-spektroskopisch bereits als sehr sauberes Produkt erwies. Die freie Base wird nach Ionenaustauschchromatographie (DOWEX 50 x W 8) erhalten. Nach Einrotieren der ammoniakalischen Lösung liegt ein Öl vor, aus dem das Produkt nach Zusatz von Toluol und erneutem Einrotieren als weißer Feststoff (110 mg)gewonnen wird.

Ausbeute: 93 %
Schmp.: 118.9 °C
Drehwert: $[\alpha]_D^{20}$ = -2.5° (c = 1.2, $CHCl_3$).

b) N-Hydroxy-L-valin-N-methylamid-hydrochlorid

100 mg (0.35 mmol) (Ξ)-(1S,2'S,5'R)-1-(N-Methyl-1-carbamoyl)-2-methyl-propyl-(2'-isopropyl-5'-methyl)-cyclohexyliden-amin-N-oxid werden in 2 ml 1 N HCl aufgenommen und bei Raumtemperatur über Nacht stehengelassen. Nach dem Eindampfen im Vakuum, wobei auch das abgespaltene Menthon ausgetrieben wird, nimmt man den glasigen Feststoff erneut in wenig Wasser auf und lyophilisiert das Produkt. Man erhält 63 mg Produkt als weißen Feststoff.
Ausbeute: 97 %

c) Das N-Hydroxy-L-aminobuttersäureamid wurde in 94 % Ausbeute durch analoge Reaktionsführung erhalten.

## Patentansprüche

**1.** Verfahren zur Herstellung von optisch aktiven Aminosäuren und Aminosäure-Derivaten der allgemeinen Formel I oder eines Säureadditionssalzes hiervon

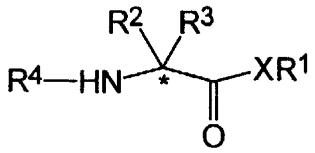

I,

worin

* = Asymmetriezentrum
X = O oder NH
$R^1$ = H, ($C_1$-$C_6$) Alkyl, Benzyl oder ($C_1$-$C_4$) Alkoxycarbonylmethyl und
$R^2$, $R^3$, unabhängig voneinander, H, ($C_1$-$C_6$) Alkyl, das mit Heteroatomen wie N, P, O, S oder Si unterbrochen oder substituiert sein kann, wobei die Heteroatome selbst ein- oder mehrfach mit ($C_1$-$C_3$) Alkyl substituiert sein können, ($C_2$-$C_6$) Alkenyl, ($C_1$-$C_6$) Haloalkyl, Halogen, Aryl, wie Naphthyl oder Phenyl, das ein- oder mehrfach mit ($C_1$-$C_3$) Alkyl, Hydroxy, Halogen oder ($C_1$-$C_3$) Alkoxy substituiert sein kann, Aralkyl wie 2-Naphthylmethyl oder Benzyl, das seinerseits ein- oder mehrfach mit ($C_1$-$C_3$) Alkyl, Hydroxy, Halogen oder ($C_1$-$C_3$) Alkoxy substituiert sein kann, Heteroaralkyl wie N-geschütztes 3-Indolylmethyl und
$R^4$ =H bedeuten,

wobei gilt, wenn $R^2$ = H ist, ist $R^3 \neq$ H,
**dadurch gekennzeichnet**,
daß man eine allgemeine Verbindung der Formel II

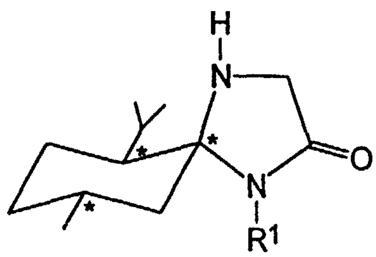

II

worin *, $R^1$ die oben angegebene Bedeutung besitzen,

a) durch Oxidation in eine Verbindung der allgemeinen Formel III

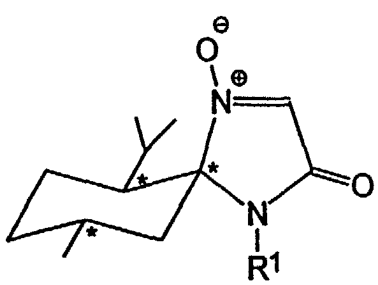

III

worin *, $R^1$ die oben angegebene Bedeutung besitzt,
überführt und

b) die Verbindungen der allgemeinen Formel III mit einem Nucleophil zu Verbindungen der allgemeinen Formel IV

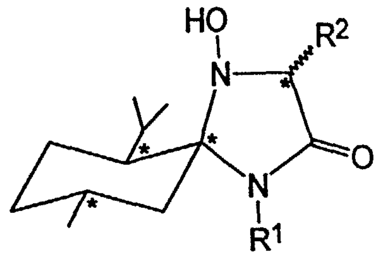

IV.

worin *, $R^1$ und $R^2$ die oben angegebene Bedeutung besitzen, umsetzt und

c) die Verbindungen der allgemeinen Formel IV zu den Verbindungen der allgemeinen Formel V

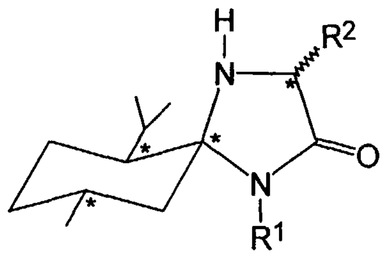

V,

worin *, $R^1$ und $R^2$ die oben angegebene Bedeutung besitzen, reduziert und

d) anschließend die Verbindungen der allgemeinen Formel V zu den L-α-Aminosäuren bzw. L-α-Aminosäure-Derivaten oder D-α-Aminosäuren bzw. D-α-Aminosäure-Derivaten der allgemeinen Formel I oder einem Säureadditionssalz hiervon hydrolysiert oder daß man die Verfahrensschritte a) und b) durchführt und anschließend

e) die Verbindung der allgemeinen Formel IV zu Verbindungen der allgemeinen Formel VI

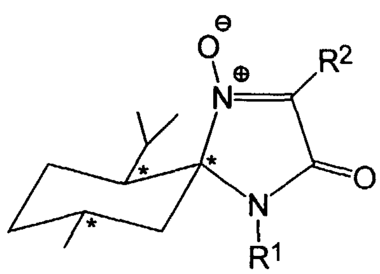

VI,

worin *, $R^1$ und $R^2$ die bereits angegebene Bedeutung besitzen, oxidiert und VI durch Umsetzung mit einem Nucleophil in Verbindungen der allgemeinen Formel VII

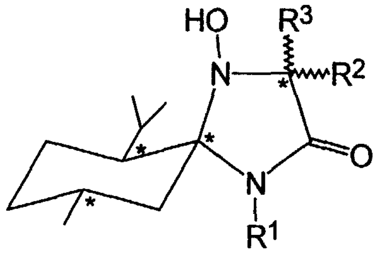

VII,

worin *, $R^1$, $R^2$ und $R^3$ die bereits angegebene Bedeutung besitzen, überführt und

f) Verbindungen der allgemeinen Formel VII zu Verbindungen der allgemeinen Formel VIII

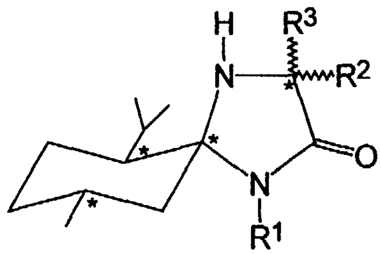

VIII,

worin *, $R^1$, $R^2$ und $R^3$ die oben angegebene Bedeutung besitzen, reduziert und anschließend

g) zu den α,α-Dialkylaminosäuren bzw. α,α-Dialkylaminosäure-Derivaten der allgemeinen Formel I oder einem Säureadditionssalz hiervon hydrolysiert.

**2.** Verfahren nach Anspruch 1,
**dadurch gekennzeichnet**,
daß

$R^1$ = ($C_1$-$C_4$) Alkyl oder Benzyl,
$R^2$, $R^3$, unabhängig voneinander, H, ($C_1$-$C_6$) Alkyl, das mit Heteroatomen wie N, P, O, S oder Si unterbrochen oder substituiert sein kann, wobei die Heteroatome selbst ein- oder mehrfach mit ($C_1$-$C_3$) Alkyl substituiert sein können, ($C_2$-$C_6$) Alkenyl, ($C_1$-$C_6$) Haloalkyl, Aryl, wie Phenyl, das ggf. ein- oder mehrfach durch Halogen substituiert sein kann, Aralkyl wie Benzyl, das seinerseits ein- oder mehrfach mit ($C_1$-$C_3$) Alkyl, Hydroxy oder ($C_1$-$C_3$) Alkoxy substituiert sein kann,
$R^4$ = H bedeuten,

wobei gilt, wenn X = O ist, kann $R^1$ = H sein, und wenn $R^2$ = H ist, ist $R^3 \neq$ H.

**3.** Verfahren zur Herstellung von optisch aktiven Aminosäuren bzw. Aminosäure-Derivaten der allgemeinen Formel I oder eines Säureadditionssalzes hiervon,

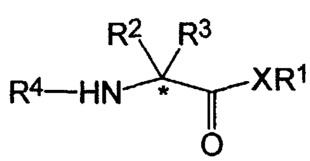

I,

worin

* = Asymmetriezentrum
X = O oder NH
$R^1$ = H, ($C_1$-$C_6$) Alkyl, Benzyl oder ($C_1$-$C_4$) Alkoxycarbonylmethyl und
$R^2$, $R^3$, unabhängig voneinander, H, ($C_1$-$C_6$) Alkyl, das mit Heteroatomen wie N, P, O, S oder Si unterbrochen oder substituiert sein kann, wobei die Heteroatome selbst ein- oder mehrfach mit ($C_1$-$C_3$) Alkyl substituiert sein können, ($C_2$-$C_6$) Alkenyl, ($C_1$-$C_6$) Haloalkyl, Halogen, Aryl, wie Naphthyl oder Phenyl, das ein- oder mehrfach mit ($C_1$-$C_3$) Alkyl, Hydroxy, Halogen oder ($C_1$-$C_3$) Alkoxy substituiert sein kann, Aralkyl wie 2-Naphthylmethyl oder Benzyl, das seinerseits ein- oder mehrfach mit ($C_1$-$C_3$) Alkyl, Hydroxy, Halogen oder ($C_1$-$C_3$) Alkoxy substituiert sein kann, Heteroaralkyl wie N-geschütztes 3-Indolylmethyl und
$R^4$ = H bedeuten,

wobei gilt, wenn $R^2$ = H ist, ist $R^3 \neq$ H,
**dadurch gekennzeichnet,**
daß man

a) die Verbindungen der allgemeinen Formeln IX und X

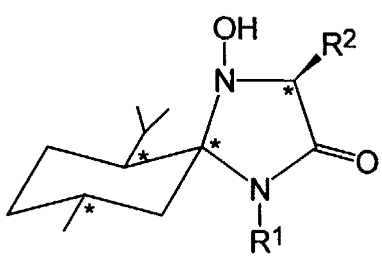

IX

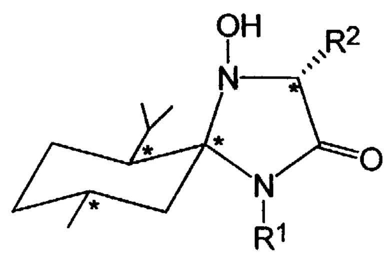

X,

worin *, $R^1$ und $R^2$ die bereits angegebene Bedeutung besitzen, und welche aus dem Nitron III

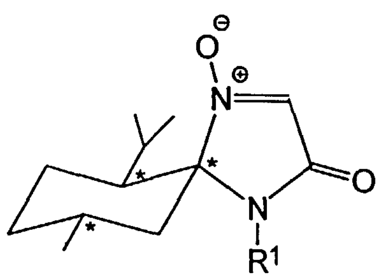

III

hergestellt werden,
b) zu Verbindungen der allgemeinen Formel XI

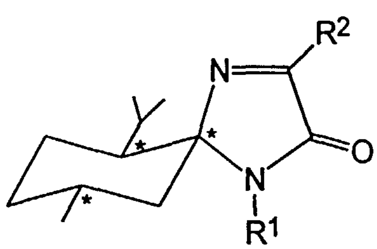

XI

worin *, $R^1$ und $R^2$ die bereits angegebene Bedeutung besitzen, dehydratisiert und anschließend die Verbindungen der allgemeinen Formel XI reduzierend invertiert, so daß aus Verbindungen des Typs IX letztlich Verbindungen des Typs XII und aus Verbindungen des Typs X letztlich Verbindungen des Typs XIII werden,

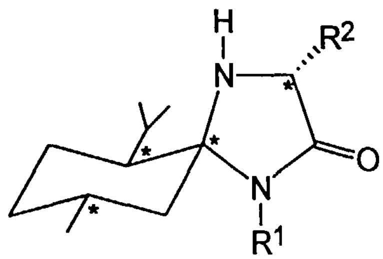

XII

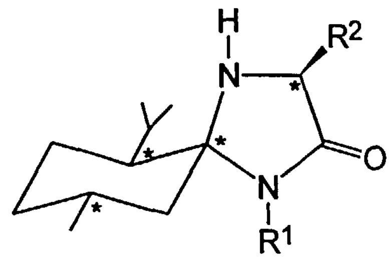

XIII

bevor man die Verbindungen der allgemeinen Formeln XII und XIII zu den Aminosäuren bzw. Aminosäure-Derivaten der allgemeinen Formel I oder einem Säureadditionssalz hiervon hydrolysiert.

**4.** Verfahren zur Herstellung von optisch aktiven N-Hydroxy-Aminosäuren der allgemeinen Formel I

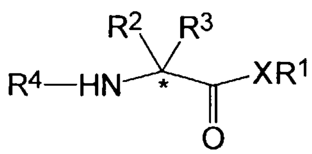

I,

worin

* = Asymmetriezentrum
X = O oder NH
$R^1$ = H, ($C_1$-$C_6$) Alkyl, Benzyl oder ($C_1$-$C_4$) Alkoxycarbonylmethyl und
$R^2$, $R^3$, unabhängig voneinander, H, ($C_1$-$C_6$) Alkyl, das mit Heteroatomen wie N, P, O, S oder Si unterbrochen oder substituiert sein kann, wobei die Heteroatome selbst ein- oder mehrfach mit ($C_1$-$C_3$) Alkyl substituiert sein können, ($C_2$-$C_6$) Alkenyl, ($C_1$-$C_6$) Haloalkyl, Halogen, Aryl, wie Naphthyl oder Phenyl, das ein- oder mehrfach mit ($C_1$-$C_3$) Alkyl, Hydroxy, Halogen oder ($C_1$-$C_3$) Alkoxy substituiert sein kann, Aralkyl wie 2-Naphthylmethyl oder Benzyl, das seinerseits ein- oder mehrfach mit ($C_1$-$C_3$) Alkyl, Hydroxy, Halogen oder ($C_1$-$C_3$) Alkoxy substituiert sein kann, Heteroaralkyl wie N-geschütztes 3-Indolylmethyl und
$R^4$ =OH bedeuten,

wobei gilt, wenn $R^2$ = H ist, ist $R^3 \neq$ H,
**dadurch gekennzeichnet**,
daß man Verbindungen der allgemeinen Formel IV

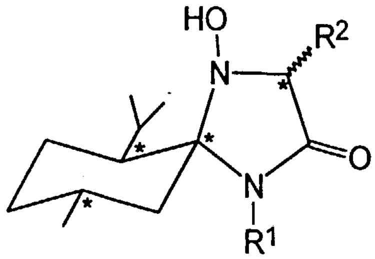

IV

welche aus dem Nitron III

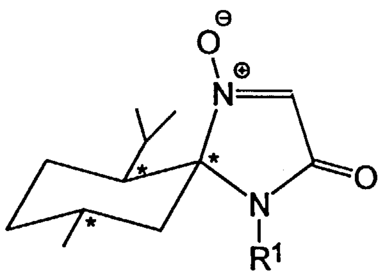

III

hergestellt werden,
mit einem metallorganischen Reagenz, ggf. in Gegenwart eines Lösungsmittels, zu Verbindungen der Formel XIV

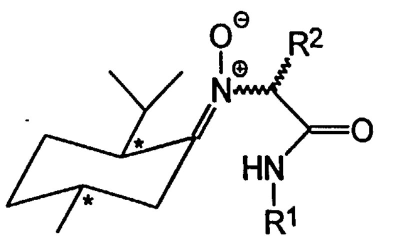

XIV

umsetzt und XIV anschließend, ggf. in Gegenwart eines Lösungsmittels und ggf. in Gegenwart einer Säure, zu Verbindungen des Typs I weiterreagieren läßt.

**5.** Verfahren nach einem der vorangegangenen Ansprüche, **dadurch gekennzeichnet**, daß die Umsetzungen zu den allgemeinen Verbindungen des Strukturtyps II in Gegenwart eines Wasserbindemittels durchgeführt werden.

**6.** Verfahren nach einem der vorangegangenen Ansprüche, **dadurch gekennzeichnet**, daß die Umsetzungen der Verbindungen des Strukturtyps III zu den Verbindungen des Strukturtyps IV in Diethylether, Tetrahydrofuran oder Toluol durchgeführt werden.

**7.** Verfahren nach einem der vorangegangenen Ansprüche, **dadurch gekennzeichnet**, daß die Umsetzungen der Verbindungen des Strukturtyps III zu den Verbindungen des Strukturtyps IV bei Temperaturen von -20 °C bis -80 °C, vorzugsweise -40 °C bis -60 °C, besonders bevorzugt bei -50 °C, durchgeführt werden.

**8.** Verfahren nach einem der vorangegangenen Ansprüche,
**dadurch gekennzeichnet**,
daß die Umsetzungen der Verbindungen des Strukturtyps IV zu den Verbindungen des Strukturtyps V als katalytische Hydrierung mit Pd oder Pt auf Aktivkohle in salzsaurer Lösung oder Ra-Ni in Alkohol erfolgen.

**9.** Verfahren nach einem der vorangegangenen Ansprüche,
**dadurch gekennzeichnet**,
daß die Umsetzungen der Verbindungen des Strukturtyps VI zu den Verbindungen des Strukturtyps VII bei Temperaturen von +80 °C bis -50 °C, vorzugsweise +25 °C bis -25 °C, besonders bevorzugt bei 0 °C, durchgeführt werden.

**10.** Verfahren gemäß Anspruch 9,
**dadurch gekennzeichnet**,
daß Toluol als Lösungsmittel verwendet wird.

**11.** Verfahren nach einem der vorhergehenden Ansprüche,
**dadurch gekennzeichnet**,
daß man Verbindungen des Typs III

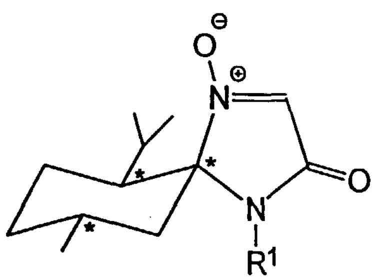

III

unter Zugabe einer Carbonsäure der Formel

$$R^2CO_2H,$$

wobei $R^2$ die oben genannte Bedeutung besitzt, in Gegenwart eines Radikalstarters und ggf. in Gegenwart eines inerten organischen Lösungsmittels direkt in Verbindungen des Typs VI

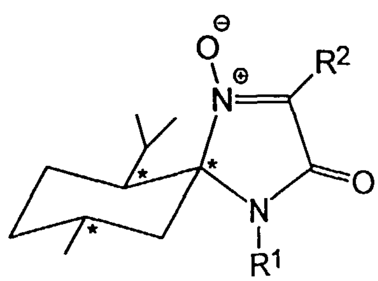

VI

überführt.

**12.** Verfahren nach Anspruch 11,
**dadurch gekennzeichnet**,
daß als Radikalstarter Dibenzoyldiperoxid, Azobisisobutyronitril, $K_2S_2O_8$/$AgNo_3$ oder $PhI(CF_3CO_2)_2$ verwendet wird.

**13.** Verfahren nach Anspruch 11,
**dadurch gekennzeichnet**,

daß als organisches Lösungsmittel ein aromatischer Kohlenwasserstoff, wie Benzol, Toluol, Xylol, Chlorbenzol oder Nitrobenzol verwendet wird.

**14.** Verfahren nach einem der vorangegangenen Ansprüche,
**dadurch gekennzeichnet**,
daß man Verbindungen des Typs V

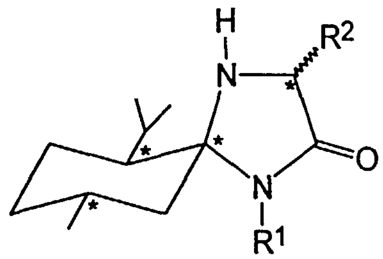

V

erhält, indem man ein Aminosäurederivat der Formel XXII

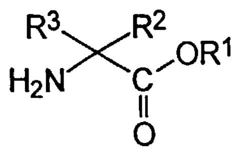

XXII

wobei $R^1$, $R^2$ und $R^3$ die bereits angegebene Bedeutung besitzen,
oder ein Säureadditionssalz hiervon, in Gegenwart einer Aminkomponente und ggf. in Gegenwart eines Lösungsmittels mit D- oder L-Menthon umsetzt.

**15.** Verfahren nach Anspruch 14,
**dadurch gekennzeichnet**,
daß als organisches Lösungsmittel ein Alkohol, wie Methanol, Ethanol, Isopropanol, n-Butanol, tert.Butanol oder sec.Butanol, verwendet wird.

**16.** Verfahren nach Anspruch 14 oder einem anderen der vorangegangenen Ansprüche,
**dadurch gekennzeichnet**,
daß als Aminkomponente Methylamin, Ethylamin, Propylamin, Benzylamin oder Isopropylamin eingesetzt wird.

**17.** Verfahren zur Herstellung von cis-4-Hydroxyprolinvorstufen,
**dadurch gekennzeichnet**,
daß man Verbindungen des Typs III

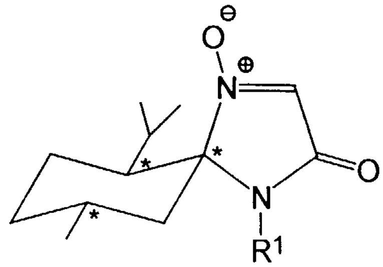

III,

worin
$R^1$ = H, ($C_1$-$C_6$) Alkyl, Benzyl oder ($C_1$-$C_4$) Alkoxycarbonylmethyl bedeutet,

a) mit einem geschützten Acroleinderivat in einer 1,3-dipolaren Cycloaddition zu Verbindungen des Typs XVI

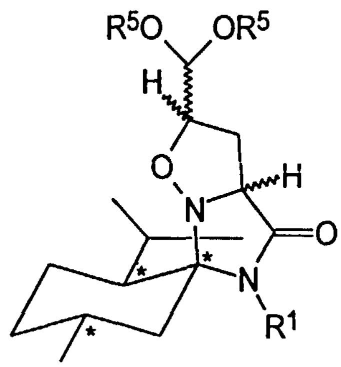

XVI

wobei $R^5$ = $CH_3$, $CH_2CH_3$ oder miteinander zu einem fünfgliedrigen Heterocyclus verbunden sein können, umsetzt,

b) anschließend zu Verbindungen des Typs XVII

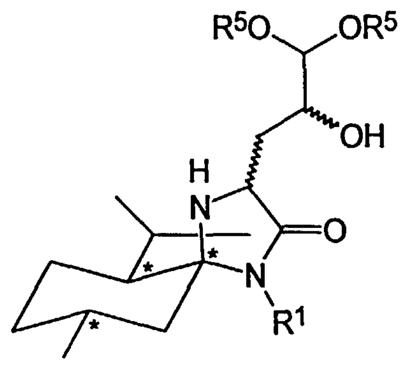

XVII

reduziert, bevor man XVII ggf. in Gegenwart einer Säure und ggf. in Gegenwart eines Lösungsmittels hydrolysiert und anschließend zu Verbindungen des Typs XVIII

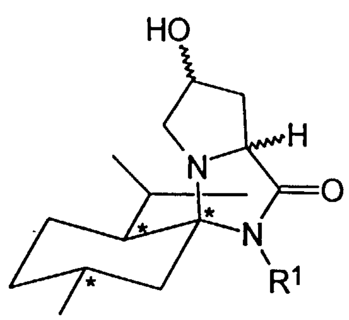

XVIII

abermals reduziert, ggf. in Gegenwart eines organischen Lösungsmittels.

**18.** Verfahren zur Herstellung von cis-4-Hydroxyprolinvorstufen,
**dadurch gekennzeichnet**,
daß man Verbindungen des Typs III

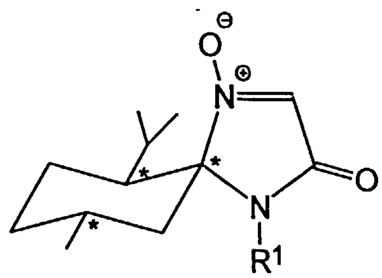

III

worin

$R^1$ = H, ($C_1$-$C_6$) Alkyl, Benzyl oder ($C_1$-$C_4$) Alkoxycarbonylmethyl bedeutet,

mit Allylalkohol, ggf. in einem organischen Lösungsmittel, zu Verbindungen des Typs XIX

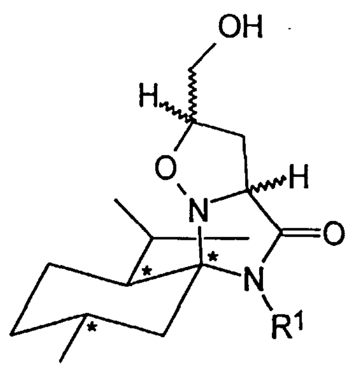

XIX

umsetzt, bevor man zu Verbindungen des Typs V

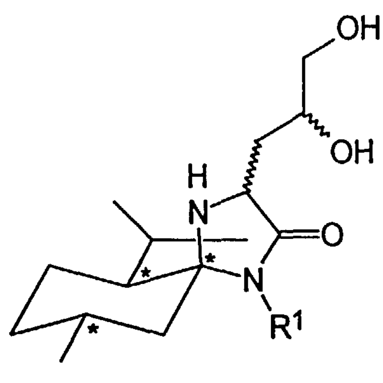

Typ V

reduziert und anschließend durch saure Hydrolyse wahlweise in Verbindungen des Typs XX und XXI

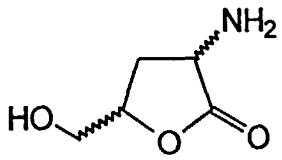

XX

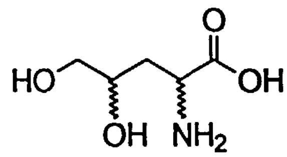

XXI

überführt.

**19.** Verbindungen des Typs XVI

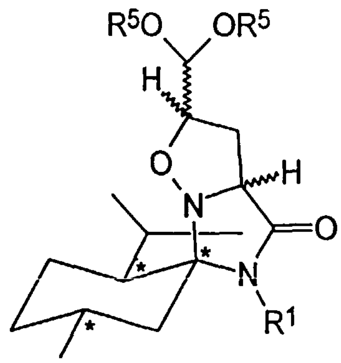

XVI

worin

* ein Asymmetriezentrum und
$R^1$ = H, ($C_1$-$C_6$) Alkyl, Benzyl oder ($C_1$-$C_4$) Alkoxycarbonylmethyl bedeutet und
$R^5$ = $CH_3$, $CH_2CH_3$ oder miteinander zu einem fünfgliedrigen Ring verbunden sein kann,

bedeutet.

**20.** Verbindungen des Typs XVIII

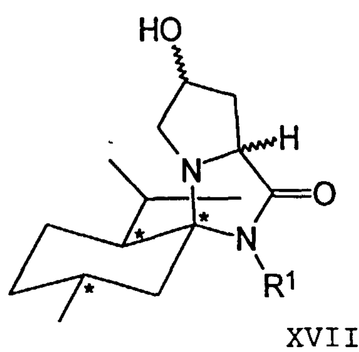

XVIII

worin

* ein Asymmetriezentrum und

$R^1$ = H, ($C_1$-$C_6$) Alkyl, Benzyl oder ($C_1$-$C_4$) Alkoxycarbonylmethyl bedeutet.

**21.** Verbindungen des Typs XIX

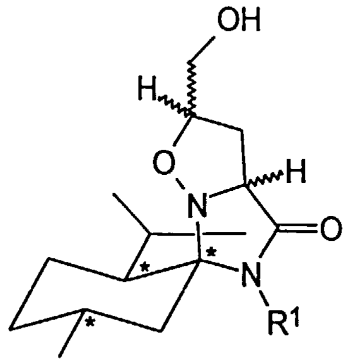

XIX

worin

* ein Asymmetriezentrum und
$R^1$ = H, ($C_1$-$C_6$) Alkyl, Benzyl oder ($C_1$-$C_4$) Alkoxycarbonylmethyl bedeutet.

## Claims

**1.** Process for the preparation of optically active amino acids and amino acid derivatives of the general formula I or of an acid addition salt thereof

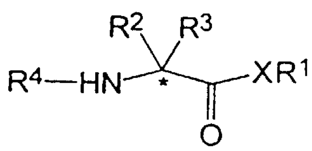

I,

wherein

* = centre of asymmetry
X = O or NH
$R^1$ = H, ($C_1$-$C_6$) alkyl, benzyl or ($C_1$-$C_4$) alkoxycarbonylmethyl and
$R^2$, $R^3$, independently of one another, denote ($C_1$-$C_6$) alkyl, which can be interrupted or substituted by heteroatoms, such as N, P, O, S or Si, it being possible for the heteroatoms themselves to be substituted by ($C_1$-$C_3$) alkyl once or several times, ($C_2$-$C_6$) alkenyl, ($C_1$-$C_6$) haloalkyl, halogen, aryl, such as naphthyl or phenyl, which can be substituted by ($C_1$-$C_3$) alkyl, hydroxyl, halogen or ($C_1$-$C_3$) alkoxy once or several times, aralkyl, such as 2-naphthylmethyl or benzyl, which in its turn can be substituted by ($C_1$-$C_3$) alkyl, hydroxyl, halogen or ($C_1$-$C_3$) alkoxy once or several times, heteroaralkyl, such as N-protected 3-indolylmethyl, and
$R^4$ = H,

wherein if $R^2$ = H, $R^3 \neq$ H,
characterized in that
a general compound of the formula II

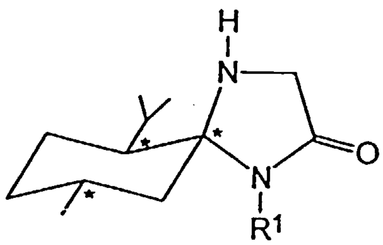

II

wherein *, $R^1$ have the abovementioned meaning,

a) is converted by oxidation into a compound of the general formula III

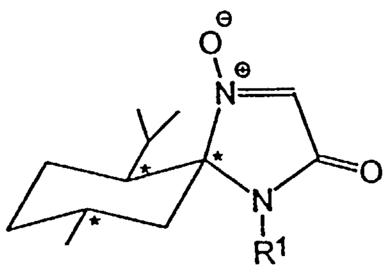

III

wherein *, $R^1$ have the abovementioned meaning, and

b) the compounds of the general formula III are reacted with a nucleophile to give compounds of the general formula IV

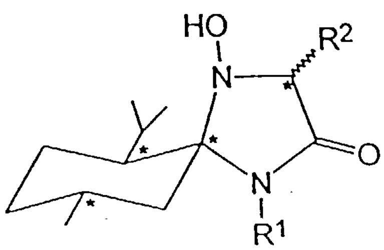

IV

wherein *, $R^1$ and $R^2$ have the abovementioned meaning, and

c) the compounds of the general formula IV are reduced to give the compounds of the general formula V

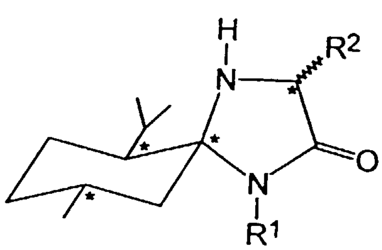

V,

wherein *, $R^1$ and $R^2$ have the abovementioned meaning,

and

d) the compounds of the general formula V are then hydrolysed to give the L-α-amino acids or L-α-amino acid derivatives or D-α-amino acids or D-α-amino acid derivatives of the general formula I or an acid addition salt thereof, or in that process steps a) and b) are carried out and then

e) the compound of the general formula IV is oxidized to give compounds of the general formula VI

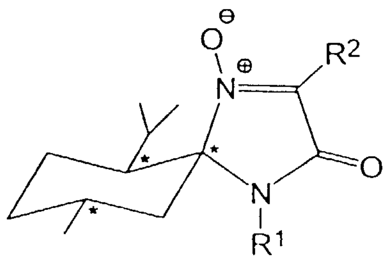

VI,

wherein *, $R^1$ and $R^2$ have the meaning already given,
and VI is converted by reaction with a nucleophile into compounds of the general formula VII

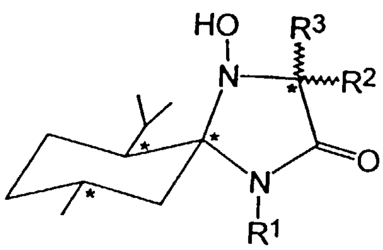

VII,

wherein *, $R^1$, $R^2$ and $R^3$ have the meaning already given,
and

f) compounds of the general formula VII are reduced to give compounds of the general formula VIII

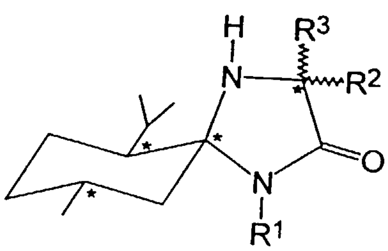

VIII,

wherein *, $R^1$, $R^2$ and $R^3$ have the abovementioned meaning,
and then

g) these are hydrolysed to give the α,α-dialkylamino acids or α,α-dialkylamino acid derivatives of the general formula I or an acid addition salt thereof.

**2.** Process according to claim 1,
characterized in that

$R^1$ = $(C_1-C_4)$ alkyl or benzyl,
$R^2$, $R^3$, independently of one another, denote H, $(C_1-C_6)$ alkyl, which can be interrupted or substituted by

heteroatoms, such as N, P, O, S or Si, it being possible for the heteroatoms themselves to be substituted by $(C_1-C_3)$ alkyl once or several times, $(C_2-C_6)$ alkenyl, $(C_1-C_6)$ haloalkyl, aryl, such as phenyl, which can optionally be substituted by halogen once or several times, aralkyl, such as benzyl, which in its turn can be substituted by $(C_1-C_3)$ alkyl, hydroxyl or $(C_1-C_3)$ alkoxy once or several times,
$R^4$ = H,

wherein if X = O, $R^1$ can = H, and if $R^2$ = H, $R^3 \neq$ H.

**3.** Process for the preparation of optically active amino acids or amino acid derivatives of the general formula I or of an acid addition salt thereof,

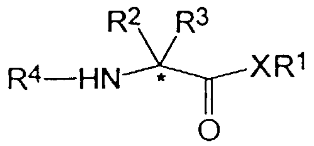
I,

wherein

* = centre of asymmetry
X = O or NH
$R^1$ = H, $(C_1-C_6)$ alkyl, benzyl or $(C_1-C_4)$ alkoxycarbonylmethyl and
$R^2$, $R^3$, independently of one another, denote H, $(C_1-C_6)$ alkyl, which can be interrupted or substituted by heteroatoms, such as N, P, O, S or Si, it being possible for the heteroatoms themselves to be substituted by $(C_1-C_3)$ alkyl once or several times, $(C_2-C_6)$ alkenyl, $(C_1-C_6)$ haloalkyl, halogen, aryl, such as naphthyl or phenyl, which can be substituted by $(C_1-C_3)$ alkyl, hydroxyl, halogen or $(C_1-C_3)$ alkoxy once or several times, aralkyl, such as 2-naphthylmethyl or benzyl, which in its turn can be substituted by $(C_1-C_3)$ alkyl, hydroxyl, halogen or $(C_1-C_3)$ alkoxy once or several times, heteroaralkyl, such as N-protected 3 -indolylmethyl, and
$R^4$ = H,

wherein if $R^2$ = H, $R^3 \neq$ H,
characterized in that

a) the compounds of the general formulae IX and X

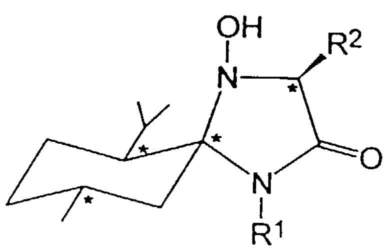
IX

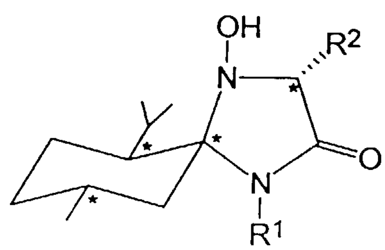
X,

wherein *, $R^1$ and $R^2$ have the meaning already given and which are prepared from the nitrone III

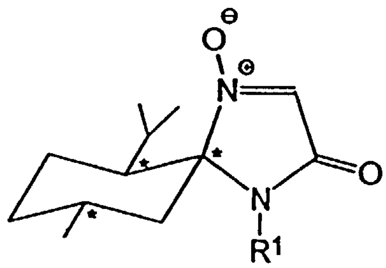

III

b) are dehydrated to give compounds of the general formula XI

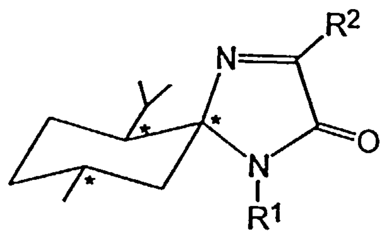

XI

wherein *, $R^1$ and $R^2$ have the meaning already given,
and the compounds of the general formula XI are then inverted by reduction, so that finally compounds of the type XII arise from compounds of the type IX and finally compounds of the type XIII arise from compounds of the type X,

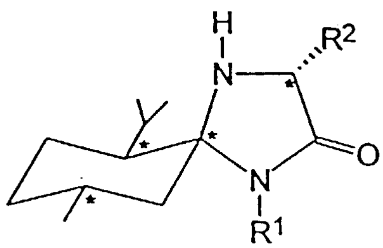

XII

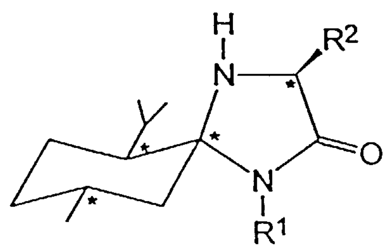

XIII

before the compounds of the general formulae XII and XIII are hydrolysed to give the amino acids or amino acid derivatives of the general formula I or an acid addition salt thereof.

**4.** Process for the preparation of optically active N-hydroxy-amino acids of the general formula I

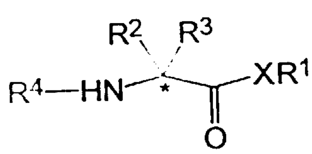

I,

wherein

* = centre of asymmetry
X = O or NH
$R^1$ = H, ($C_1$-$C_6$) alkyl, benzyl or ($C_1$-$C_4$) alkoxycarbonylmethyl and
$R^2$, $R^3$, independently of one another, denote H, ($C_1$-$C_6$) alkyl, which can be interrupted or substituted by heteroatoms, such as N, P, O, S or Si, it being possible for the heteroatoms themselves to be substituted by ($C_1$-$C_3$) alkyl once or several times, ($C_2$-$C_6$) alkenyl, ($C_1$-$C_6$) haloalkyl, halogen, aryl, such as naphthyl or phenyl, which can be substituted by ($C_1$-$C_3$) alkyl, hydroxyl, halogen or ($C_1$-$C_3$) alkoxy once or several times, aralkyl, such as 2-naphthylmethyl or benzyl, which in its turn can be substituted by ($C_1$-$C_3$) alkyl, hydroxyl, halogen or ($C_1$-$C_3$) alkoxy once or several times, heteroaralkyl, such as N-protected 3-indolylmethyl, and
$R^4$ = OH,

wherein if $R^2$ = H, $R^3 \neq$ H,
characterized in that
compounds of the general formula IV

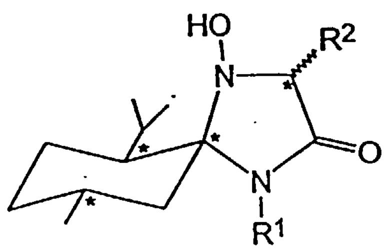

IV

which are prepared from the nitrone III

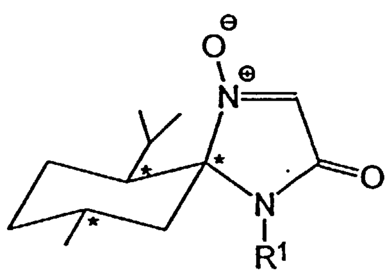

III

are reacted with an organometallic reagent, optionally in the presence of a solvent, to give compounds of the formula XIV

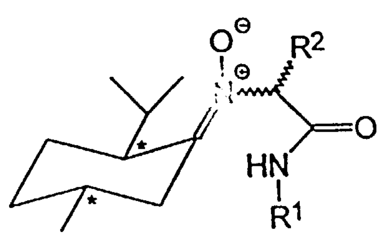

XIV

and XIV is then further reacted, optionally in the presence of a solvent and optionally in the presence of an acid, to give compounds of the type I.

5. Process according to one of the preceding claims,

characterized in that
the reactions to give the general compounds of the structure type II are carried out in the presence of a water-binding agent.

**6.** Process according to one of the preceding claims,
characterized in that
the reactions of the compounds of the structure type III to give compounds of the structure type IV are carried out in diethyl ether, tetrahydrofuran or toluene.

**7.** Process according to one of the preceding claims,
characterized in that
the reactions of the compounds of the structure type III to give the compounds of the structure type IV are carried out at temperatures from -20 °C to 80 °C, preferably -40 °C to -60 °C, particularly preferably at -50 °C.

**8.** Process according to one of the preceding claims,
characterized in that
the reactions of the compounds of the structure type IV to give the compounds of the structure type V are carried out as catalytic hydrogenation with Pd or Pt on active charcoal in hydrochloric acid solution or Ra-Ni in alcohol.

**9.** Process according to one of the preceding claims,
characterized in that
the reactions of the compounds of the structure type VI to give the compounds of the structure type VII are carried out at temperature from +80 °C to -50 °C, preferably +25 °C to -25 °C, particularly preferably at 0 °C.

**10.** Process according to claim 9,
characterized in that
toluene is used as the solvent.

**11.** Process according to one of the preceding claims,
characterized in that
compounds of the type III

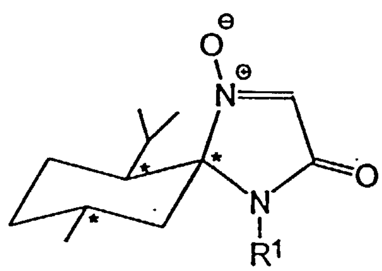

III

are converted directly, with addition of a carboxylic acid of the formula

$$R^2CO_2H,$$

wherein $R^2$ has the abovementioned meaning, in the presence of a free-radical initiator and optionally in the presence of an inert organic solvent, into compounds of the type VI

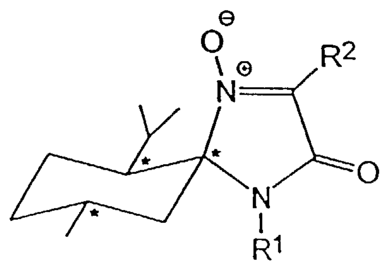

VI

12. Process according to claim 11, characterized in that dibenzoyl diperoxide, azobisisobutyronitrile, $K_2S_2O_8$/$AgNo_3$ or $PhI(CF_3CO_2)_2$ is used as the free-radical initiator.

13. Process according to claim 11, characterized in that an aromatic hydrocarbon, such as benzene, toluene, xylene, chlorobenzene or nitrobenzenze, is used as the organic solvent.

14. Process according to one of the preceding claims, characterized in that compounds of the type V

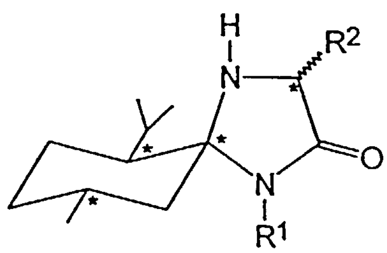

V

are obtained by a procedure in which an amino acid derivative of the formula XXII

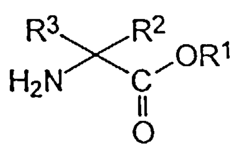

XXII

wherein $R^1$, $R^2$ and $R^3$ have the meaning already given, or an acid addition salt thereof, is reacted with D- or L-menthone in the presence of an amine component and optionally in the presence of a solvent.

15. Process according to claim 14, characterized in that an alcohol, such as methanol, ethanol, isopropanol, n-butanol, tert.butanol or sec.butanol, is used as the organic solvent.

16. Process according to claim 14 or another of the preceding claims, characterized in that methylamine, ethylamine, propylamine, benzylamine or isopropylamine is employed as the amine component.

17. Process for the preparation of cis-4-hydroxyproline precursors,

characterized in that
compounds of the type III

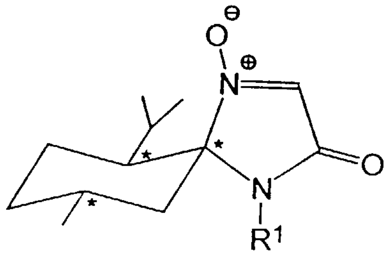

III,

wherein

$R^1$ = H, $(C_1-C_6)$ alkyl, benzyl or $(C_1-C_4)$ alkoxycarbonylmethyl,

a) are reacted with a protected acrolein derivative in a 1,3-dipolar cycloaddition to give compounds of the type XVI

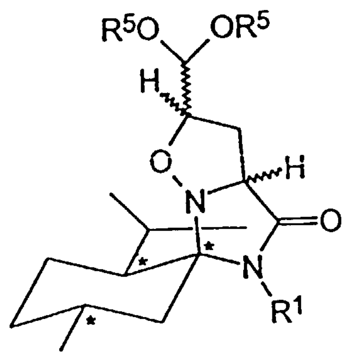

XVI

wherein $R^5$ = $CH_3$, $CH_2CH_3$ or can be joined to one another to give a five-membered heterocyclic ring,

b) these are then reduced to give compounds of the type XVII

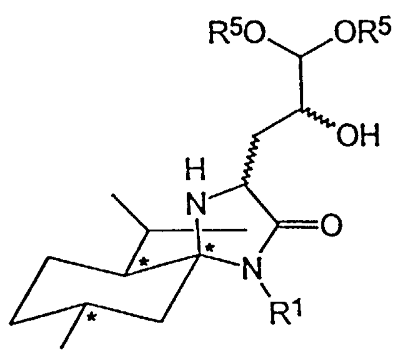

XVII

before XVII is hydrolysed, optionally in the presence of an acid and optionally in the presence of a solvent, and then reduced again to give compounds of the type XVIII

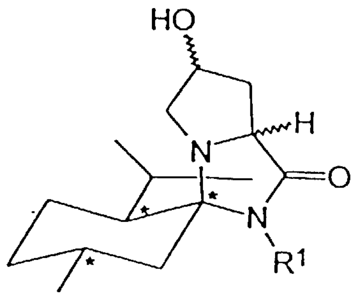

XVIII

optionally in the presence of an organic solvent.

**18.** Process for the preparation of cis-4-hydroxyproline precursors, characterized in that compounds of the type III

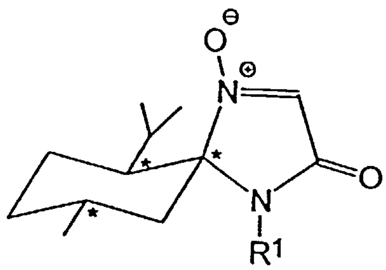

III

wherein

$R^1$ = H, ($C_1$-$C_6$) alkyl, benzyl or ($C_1$-$C_4$) alkoxycarbonylmethyl,

are reacted with allyl alcohol, optionally in an organic solvent, to give compounds of the type XIX

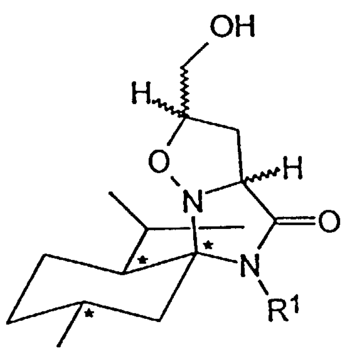

XIX

before these are reduced to give compounds of the type V

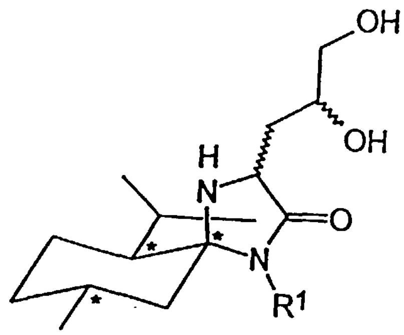

type V

which are then optionally converted by acid hydrolysis into compounds of the type XX and XXI

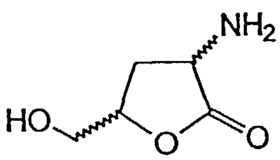

XX

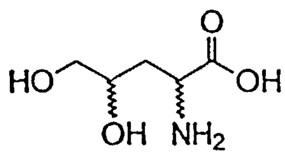

XXI

19. Compounds of the type XVI

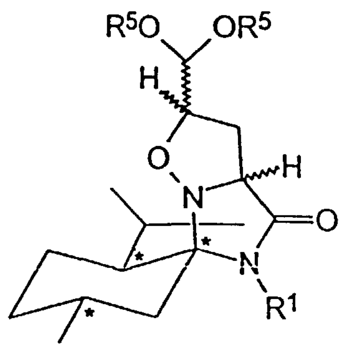

XVI

wherein

| | |
|---|---|
| * | denotes a centre of asymmetry and |
| $R^1$ = | H, ($C_1$-$C_6$) alkyl, benzyl or ($C_1$-$C_4$) alkoxycarbonylmethyl and |
| $R^5$ = | $CH_3$, $CH_2CH_3$ or can be joined to one another to give a five-membered ring. |

20. Compounds of the type XVIII

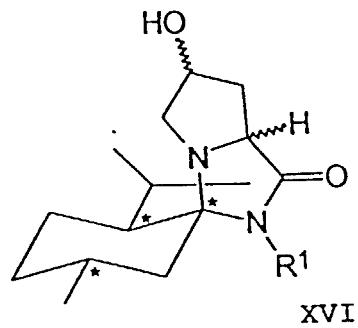

XVIII

wherein

* denotes a centre of asymmetry and
$R^1$ = H, ($C_1$-$C_6$) alkyl, benzyl or ($C_1$-$C_4$) alkoxycarbonylmethyl.

21. Compounds of the type XIX

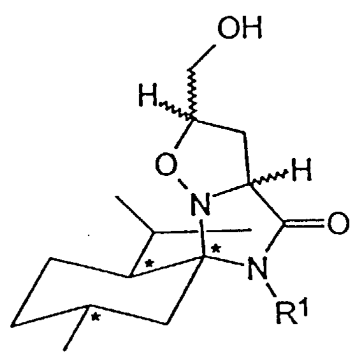

XIX

wherein

* denotes a centre of asymmetry and
$R^1$ = H, ($C_1$-$C_6$) alkyl, benzyl or ($C_1$-$C_4$) alkoxycarbonylmethyl.

**Revendications**

**1.** Procédé pour la préparation d'aminoacides et de dérives d'aminoacides optiquement actifs de formule générale I ou d'un sel d'addition acide de ceux-ci,

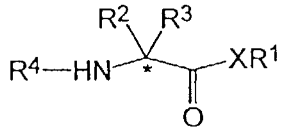

I,

dans laquelle

* est un centre d'asymétrie,

X = l'atome d'O ou un groupe NH

$R^1$ = l'atome d'H, un groupe alkyle en $C_1$ à $C_6$, un groupe benzyle ou un groupe alcoxycarbonylméthyle en $C_1$ à $C_4$ et

$R^2$, $R^3$ représentent, indépendamment l'un de l'autre, l'atome d'H, un groupe alkyle en $C_1$ à $C_6$, qui peut être interrompu ou substitué par des hétéroatomes tels que N, P, O, S ou Si, les hétéroatomes eux-mêmes pouvant être substitués une ou plusieurs fois par un groupe alkyle en $C_1$ à $C_3$, un groupe alkényle en $C_2$ à $C_6$, un groupe haloalkyle en $C_1$ à $C_6$, un atome d'halogène, un groupe aryle, tel qu'un groupe naphtyle ou phényle, qui peut être substitué une ou plusieurs fois par un groupe alkyle en $C_1$ à $C_3$, un groupe hydroxy, un atome d'halogène ou un groupe alcoxy en $C_1$ à $C_3$, un groupe aralkyle tels qu'un groupe 2-naphtylméthyle ou benzyle, qui peut, à son tour, être substitué une ou plusieurs fois par un groupe alkyle en $C_1$ à $C_3$, un groupe hydroxy, un atome d'halogène ou un groupe alcoxy en $C_1$ à $C_3$, un groupe hétéroaralkyle, tel qu'un groupe 3-indolylméthyle dont l'atome N est protégé et

$R^4$ représente l'atome d'H,

où $R^3$ est différent de l'atome d'H lorsque $R^2$ représente l'atome d'H, caractérisé en ce qu'on transforme un composé général de formule II

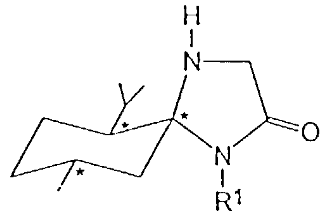

II

dans laquelle * et $R^1$ ont la signification indiquée ci-dessus,

a) par oxydation en un composé de formule générale III

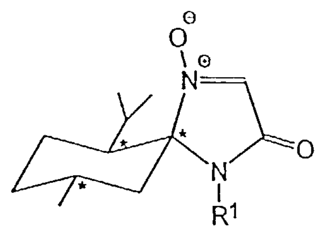

III

dans laquelle *, $R^1$ ont la signification indiquée ci-dessus, et

b) en ce qu'on transforme les composés de formule générale III avec un nucléophile en composés de formule générale IV

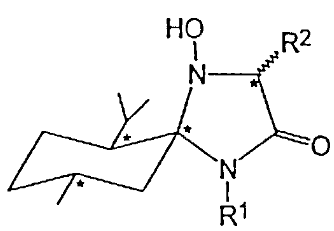

IV,

dans laquelle *, $R^1$ et $R^2$ ont la signification indiquée ci-dessus, et

c) en ce qu'on réduit les composés de formule générale IV en composés de formule générale V

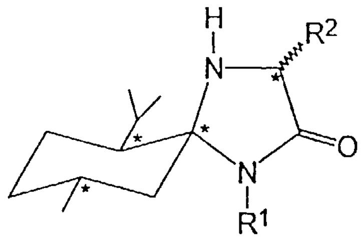

V,

dans laquelle *, $R^1$ et $R^2$ ont la signification indiquée ci-dessus, et

d) en ce qu'on hydrolyse ensuite les composés de formule générale V en L-α-aminoacides ou en dérivés de L-α-aminoacides ou en D-α-aminoacides ou en dérivés de D-α-aminoacides de formule générale I ou un sel d'addition acide de ceux-ci ou en ce qu'on réalise les étapes de procédé a) et b), puis

e) en ce qu'on oxyde le composé de formule générale IV en composés de formule générale VI

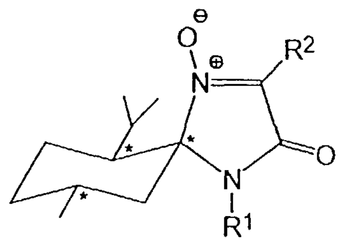

VI,

dans laquelle *, $R^1$ et $R^2$ ont la signification déjà indiquée ci-dessus, et en ce qu'on transforme le composé VI, par réaction avec un nucléophile, en composés de formule générale VII

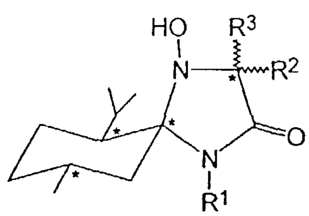

VII,

dans laquelle *, $R^1$, $R^2$ et $R^3$ ont la signification déjà indiquée ci-dessus, et

f) en ce qu'on réduit des composés de formule générale VII en composés de formule générale VIII

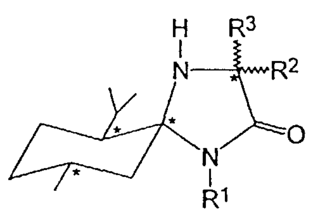

VIII,

dans laquelle *, $R^1$ et $R^2$ et $R^3$ ont la signification indiquée ci-dessus, et

g) en ce qu'on les hydrolyse ensuite en α,α-dialkylaminoacides ou dérivés de α,α-dialkylaminoacides de formule générale I ou un sel d'addition acide de ceux-ci.

**2.** Procédé selon la revendication 1, caractérisé en ce que

$R^1$ = un groupe alkyle en $C_1$ à $C_4$ ou un groupe benzyle, et

$R^2$, $R^3$ représentent, indépendamment l'un de l'autre, l'atome d'H, un groupe alkyle en $C_1$ à $C_6$, qui peut être interrompu ou substitué par des hétéroatomes tels que N, P, O, S ou Si, les hétéroatomes eux-mêmes pouvant être substitués une ou plusieurs fois par un groupe alkyle en $C_1$ à $C_3$, un groupe alkényle en $C_2$ à $C_6$, un groupe haloalkyle en $C_1$ à $C_6$, un groupe aryle, tel qu'un groupe phényle, qui peut, le cas échéant, être substitué une ou plusieurs fois par un atome d'halogène, un groupe aralkyle tel qu'une groupe benzyle, qui peut, à son tour, être substitué une ou plusieurs fois par un groupe alkyle en $C_1$ à $C_3$, un groupe hydroxy ou un groupe alcoxy en $C_1$ à $C_3$, et

$R^4$ représente l'atome d'H,

où, lorsque X représente l'atome d'O, $R^1$ peut être l'atome d'H et lorsque $R^2$ représente l'atome d'H, $R^3$ est différent de H.

**3.** Procédé pour la préparation d'aminoacides ou des dérivés d'aminoacides optiquement actifs de formule générale 1 ou d'un sel d'addition acide de ceux-ci,

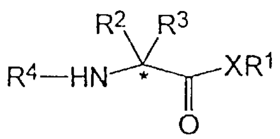

I,

dans laquelle

* est un centre d'asymétrie,

X = l'atome d'O ou un groupe NH

$R^1$ = l'atome d'H, un groupe alkyle en $C_1$ à $C_6$, un groupe benzyle ou un groupe alcoxycarbonylméthyle en $C_1$ à $C_4$ et

$R^2$, $R^3$ représentent, indépendamment l'un de l'autre, l'atome d'H, un groupe alkyle en $C_1$ à $C_6$, qui peut être interrompu ou substitué par des hétéroatomes tels que N, P, O, S ou Si, les hétéroatomes eux-mêmes pouvant être substitués une ou plusieurs fois par un groupe alkyle en $C_1$ à $C_3$, un groupe alkényle en $C_2$ à $C_6$, un groupe haloalkyle en $C_1$ à $C_6$, un atome d'halogène, un groupe aryle, tel qu'un groupe naphtyle ou phényle, qui peut être substitué une ou plusieurs fois par un groupe alkyle en $C_1$ à $C_3$, un groupe hydroxy, un atome d'halogène ou un groupe alcoxy en $C_1$ à $C_3$, un groupe aralkyle tels qu'un groupe 2-naphtylméthyle ou benzyle, qui peut, à son tour, être substitué une ou plusieurs fois par un groupe alkyle en $C_1$ à $C_3$, un groupe hydroxy, un atome d'halogène ou un groupe alcoxy en $C_1$ à $C_3$, un groupe hétéroaralkyle, tel qu'un groupe 3-indolylméthyle dont l'atome de N est protégé et

$R^4$ représente l'atome d'H,

où $R^3$ est différent de l'atome d'H lorsque $R^2$ représente l'atome d'H,
caractérisé

a) en ce qu'on déshydrate les composés de formule générale IX et X

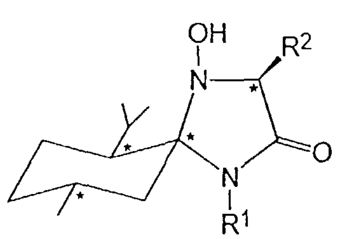

IX

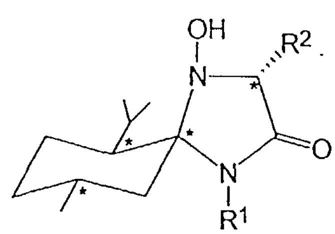

X,

dans lesquelles *, $R^1$ et $R^2$ présentant la signification déjà indiquée et qui sont préparés à partir du nitron

III

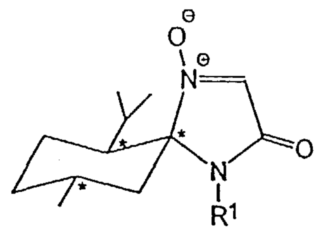

III

b) en composés de formule générale XI

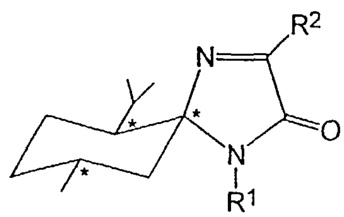

XI,

dans laquelle *, $R^1$ et $R^2$ présentent la signification déjà mentionnée

et en ce qu'on invertit ensuite les composés de formule générale XI par réduction, de telle manière qu'on obtient enfin, à partir des composés de type IX, des composés de type XII et à partir des composés de type X des composés de type XIII

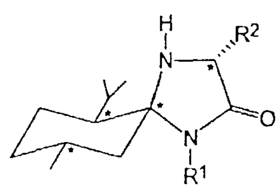

XII

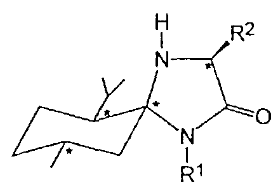

XIII

avant d'hydrolyser les composés des formules générales XII et XIII en aminoacides ou dérives d'aminoacides de formule I ou un sel d'addition acide de ceux-ci.

**4.** Procédé pour la préparation d'aminoacides ou de dérivés d'aminoacides optiquement actifs de formule générale I ou d'un sel d'addition acide de ceux-ci,

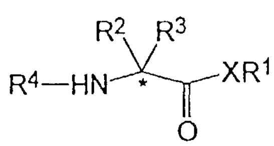

I,

dans laquelle

| | |
|---|---|
| * | est un centre d'asymétrie, |
| X = | l'atome d'O ou un groupe NH |
| $R^1$ = | l'atome d'H, un groupe alkyle en $C_1$ à $C_6$, un groupe benzyle ou un groupe alcoxycarbonylméthyle en $C_1$ à $C_4$ et |
| $R^2$, $R^3$ | représentent, indépendamment l'un de l'autre, l'atome d'H, un groupe alkyle en $C_1$ à $C_6$, qui peut être |

interrompu ou substitué par des hétéroatomes tels que N, P, O, S ou Si, les hétéroatomes eux-mêmes pouvant être substitués une ou plusieurs fois par un groupe alkyle en $C_1$ à $C_3$, un groupe alkényle en $C_2$ à $C_6$, un groupe haloalkyle en $C_1$ à $C_6$, un atome d'halogène, un groupe aryle, tel qu'un groupe naphtyle ou phényle, qui peut être substitué une ou plusieurs fois par un groupe alkyle en $C_1$ à $C_3$, un groupe hydroxy, un atome d'halogène ou un groupe alcoxy en $C_1$ à $C_3$, un groupe aralkyle tels qu'un groupe 2-naphtylméthyle ou benzyle, qui peut, à son tour, être substitué une ou plusieurs fois par un groupe alkyle en $C_1$ à $C_3$, un groupe hydroxy, un atome d'halogène ou un groupe alcoxy en $C_1$ à $C_3$, un groupe hétéroaralkyle, tel qu'un groupe 3-indolylméthyle dont l'atome N est protégé et

$R^4$ représente le groupe OH,

où $R^3$ est différent de l'atome d'H lorsque $R^2$ représente l'atome d'H,
caractérisé
en ce qu'on transforme des composés de formule générale IV

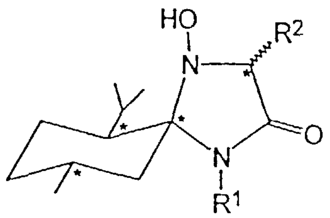

IV,

préparés à partir du nitron III

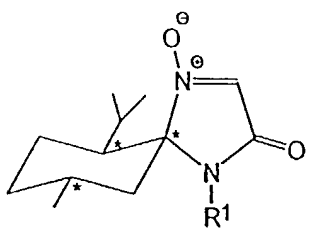

III

avec un réactif organométallique, le cas échéant en présence d'un solvant, en composés de formule XIV

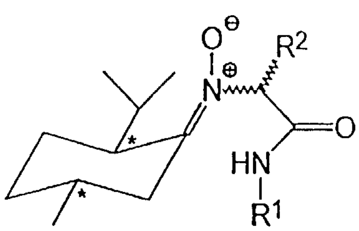

XIV

et en ce qu'on fait ensuite réagir le composé XIV, le cas échéant en présence d'un solvant et le cas échéant en présence d'un acide, en composés de type I.

**5.** Procédé selon une quelconque des revendications précédentes, caractérisé en ce que les transformations en composés généraux de type de structure II sont réalisées en présence d'un agent de fixation de l'eau.

**6.** Procédé selon une quelconque des revendications précédentes, caractérisé en ce que les transformations des composés du type de structure III en composés du type de structure IV sont effectuées dans du diéthyléther, du tétrahydrofuranne ou du toluène.

**7.** Procédé selon une quelconque des revendications précédentes, caractérisé en ce que les transformations des composés du type de structure III en composés du type de structure IV sont effectuées à des températures de -20 °C à -80 °C, de préférence de-40 °C à -60 °C, de manière particulièrement préférée à -50 °C.

**8.** Procédé selon une quelconque des revendications précédentes, caractérisé en ce que les transformations des composés du type de structure IV en composés du type de structure V sont réalisées comme hydratation catalytique avec du Pd ou du Pt sur du charbon actif dans une solution chlorhydrique ou du Ra-Ni dans de l'alcool.

**9.** Procédé selon une quelconque des revendications précédentes, caractérisé en ce que les transformations des composés du type de structure VI en composés du type de structure VII sont effectuées à des températures de +80 °C à -50 °C, de préférence de +25 °C à -25 °C, de manière particulièrement préférée à 0 °C.

**10.** Procédé selon la revendication 9, caractérisé en ce qu'on utilise du toluène comme solvant.

**11.** Procédé selon une quelconque des revendications précédentes, caractérisé en ce qu'on transforme des composés de type III

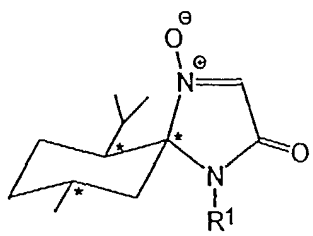

III

,

par addition d'un acide carboxylique de formule

$$R^2CO_2H$$

où $R^2$ a la signification susmentionnée, en présence d'un composé pour amorcer les radicaux et, le cas échéant, en présence d'un solvant organique interne, directement en composés de type VI

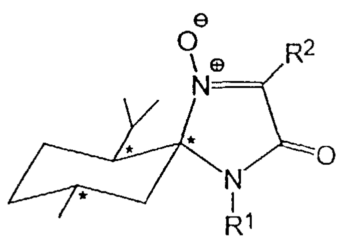

VI,

**12.** Procédé selon la revendication 11, caractérisé ce qu'on utilise comme initiateur des radicaux du diperoxyde de dibenzoyle, de l'azobisisobutyronitrile, du $K_2S_2O_8/AgNO_3$ ou du $PhI(CF_3CO_2)_2$.

**13.** Procédé selon la revendication 11, caractérisé en ce qu'on utilise comme solvant organique un hydrocarbure aromatique, tel que du benzène, du toluène, du xylène, du chlorobenzène ou du nitrobenzène.

**14.** Procédé selon une quelconque des revendications précédentes, caractérisé en ce qu'on obtient vies composés du type V

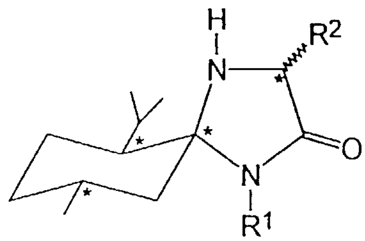

V,

lorsqu'on fait réagir un dérivé d'aminoacide de formule XXII

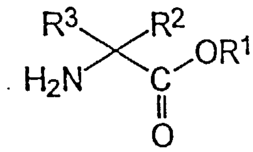

XXII

dans laquelle $R^1$, $R^2$ et $R^3$ ont la signification déjà indiquée ou un sel d'addition de celui-ci ,en présence d'une composante amine et, le cas échéant, en présence d'un solvant, avec de la D-menthone ou de la L-menthone.

**15.** Procédé selon la revendication 14, caractérisé en ce qu'on utilise comme solvant organique un alcool, tel que du méthanol, de l'éthanol, de l'isopropanol, du n-butanol, du tert-butanol ou du sec.-butanol.

**16.** Procédé selon la revendication 14 ou une quelconque autre des revendications ci-dessus, caractérisé en ce qu'on utilise comme composante amine de la méthylamine, de l'éthylamine, de la propylamine, de la benzylamine ou de l'isopropylamine.

**17.** Procédé pour la préparation d'état précurseurs de la cis-4-hydroxyproline, caractérisé en ce qu'on transforme des composés de type III

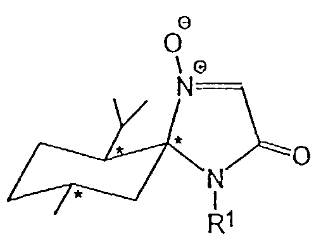

III

dans lequel

$R^1$ = l'atome d'H, un groupe alkyle en $C_1$ à $C_6$, un groupe benzyle ou un groupe alcoxycarbonylméthyle en $C_1$ à $C_4$,

a) avec un dérivé d'acroléine protégé dans une cycloaddition 1,3-dipolaire en composés du type XVI

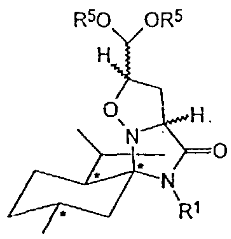

XVI

dans lequel $R^5$ représente $CH_3$, $CH_2CH_3$ ou peuvent être reliés l'un à l'autre pour former un hétérocycle à cinq liaisons,
b) en ce qu'on réduit ensuite en composés de type XVIII

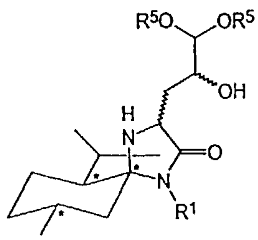

XVII

avant qu'on hydrolyse XVII, le cas échéant en présence d'un acide et le cas échéant en présence d'un solvant, et en ce qu'on réduit ensuite en composés du type XVIII

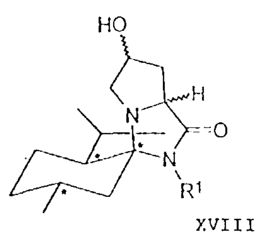

XVIII

le cas échéant en présence d'un solvant organique.

**18.** Procédé pour la préparation d'états précurseurs de cis-4-hydroxyproline, caractérisé en ce qu'on transforme des composés de type III

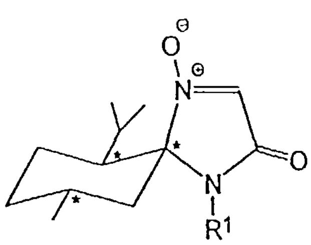

III

dans lequel
$R^1$ représente l'atome d'H, un groupe alkyle en $C_1$ à $C_6$, un groupe benzyle ou un groupe alcoxycarbonylméthyle en $C_1$ à $C_4$
avec de l'alcool allylique, le cas échéant dans un solvant organique, en composés de type XIX

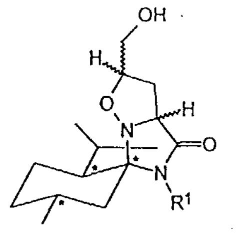

XIX

avant de les réduire en composés de type V

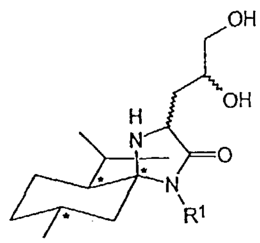

et en ce qu'on les transforme ensuite par hydrolyse acide, au choix, en composés de type XX et XXI

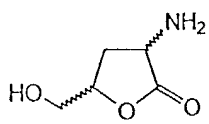

XX

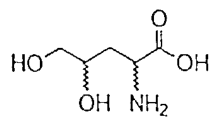

XXI

**19.** Composés du type XVI

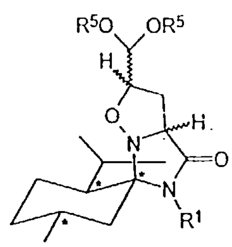

XVI

dans lequel

- * est un centre d'asymétrie,
- $R^1$ = l'atome d'H, un groupe alkyle en $C_1$ à $C_6$, un groupe benzyle ou un groupe alcoxycarbonylméthyle en $C_1$ à $C_4$ et
- $R^5$ = le groupe $CH_3$, le groupe $CH_2CH_3$, ou reliés l'un à l'autre pour former un hétérocycle à cinq liaisons.

**20.** Composés de type XVIII

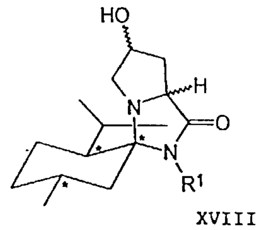

XVIII

dans lequel

- * est un centre d'asymétrie,
- $R^1$ = l'atome d'H, un groupe alkyle en $C_1$ à $C_6$, un groupe benzyle ou un groupe alcoxycarbonylméthyle en $C_1$ à $C_4$.

**21.** Composés du type XIX

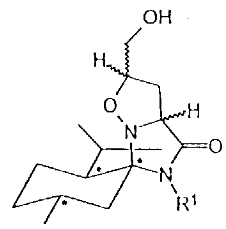

XIX

dans lequel

- * est un centre d'asymétrie,
- $R^1$ = l'atome d'H, un groupe alkyle en $C_1$ à $C_6$, un groupe benzyle ou un groupe alcoxycarbonylméthyle en $C_1$ à $C_4$.